# EUROPÄISCHE PATENTSCHRIFT

(11) **EP 0 790 824 B1**
(45) Veröffentlichungstag und Bekanntmachung des Hinweises auf die Patenterteilung: **06.02.2002**
(21) Anmeldenummer: 95939248.1
(22) Anmeldetag: 09.11.1995
(51) Int. Cl.: A61K 31/20

(54) **2,2-DICHLORALKANCARBONSÄUREN, VERFAHREN ZU IHRER HERSTELLUNG DIESE ENTHALTENDE ARZNEIMITTEL, UND IHRE VERWENDUNG ZUR BEHANDLUNG DER INSULINRESISTENZ**
2,2-DICHLOROALKANE CARBOXYLIC ACIDS, PROCESS FOR PREPARING THE SAME, MEDICAMENT CONTAINING THE SAME, AND USE THEREOF FOR TREATING INSULIN RESISTANCE
ACIDES 2,2-DICHLOROALCANECARBOXYLIQUES, LEUR PROCEDE DE PREPARATION, MEDICAMENTS LES CONTENANT, ET LEUR UTILISATION DANS LE TRAITEMENT DE LA RESISTANCE INSULINIQUE

(30) Priorität: 09.11.1994 DE 4439947
(43) Veröffentlichungstag der Anmeldung: 27.08.1997
(73) Patentinhaber: Roche Diagnostics GmbH, 68298 Mannheim (DE)
(72) Erfinder: VOSS, Edgar, D-68519 Viernheim (DE); PILL, Johannes, D-69181 Leimen (DE); FREUND, Peter, D-68775 Ketsch (DE)
(86) Internationale Anmeldenummer: EP9504413
(87) Internationale Veröffentlichungsnummer: WO9615784

(56) Entgegenhaltungen:
- EP-A- 0 081 930
- WO-A-88/02746
- WO-A-94/02128
- BULL. SOC. CHIM. BELG., 1988, 525-33, XP002000698 DE BUYCK, L. ET AL: ".alpha.,.alpha.-Dichloroaldehydes and.alpha.,.alpha.-dichlorocarboxylic acids from long chain 1-alkanols. Improved chlorination in the system DMF-chloroform-magnesium chloride"
- IND. ENG. CHEM. RES., 1992, 2425-37, XP002000699 PAATERO, ERKKI ET AL: "Selective synthesis of.alpha.-chlorocarboxylic acids"
- BULL. CHEM. SOC. JPN., 1994, 1622-6, XP002000700 BONI, MONICA ET AL: "Preparation of 2,2-dihalocarboxylic acid methyl esters by oxidation-chlorination of 2-(1-haloalkyl)-4-methyl-1,3-dioxolanes with trichloroisocyanuric acid"
- TETRAHEDRON LETT., 1994, 2961-4, XP002000701 BELLESIA, FRANCO ET AL: "Methyl.alpha.,.alpha.-dichloro-esters by oxidation-chlorination of cyclic acetals with trichloroisocyanuric acid"
- THE JOURNAL OF MEDICINAL CHEMISTRY, Bd. 32, Nr. 9, 1989, Seiten 2072-2084, XP002000702 BAR-TANA, J. ET AL: "SYNTHESIS AND HYPOLIPIDEMIC AND ANTIDIABETOGENIC ACTIVITIES OF B,B,B',B'-TETRASUBSTITUTED, LONG-CHAIN DIOIC ACIDS"

## Beschreibung

Gegenstand der vorliegenden Erfindung sind 2,2-Dichloralkancarbonsäuren, Verfahren zu deren Herstellung und Arzneimittel, die diese Verbindungen enthalten.

Die Erfindung betrifft Arzneimittel, enthaltend mindestens eine 2,2-Dichloralkancarbonsäure der allgemeinen Formel I 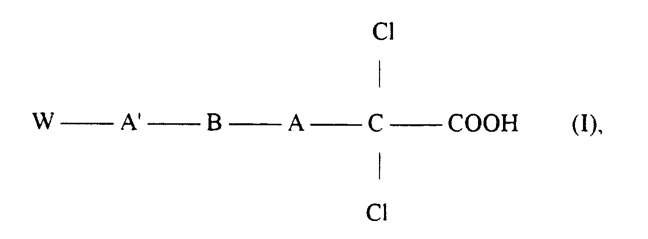 in welcher
- A: eine Alkylenkette mit 5 - 20 Kohlenstoffatomen.
- A': ein Valenzstrich, eine Vinylen- oder Acetylengruppe oder eine Alkylenkette mit 1 - 10 Kohlenstoffatomen,
- B: ein Valenzstrich, eine Methylengruppe, Schwefel, Sauerstoff oder die Gruppe NR¹,
wobei
R¹ Wasserstoff, Benzyl, Phenyl oder ein C₁-C₄-Alkylrest sein kann, eine Carbonyl-, Sulfonamid-, Sulfoxid- oder Sulfongruppe, eine E- oder Z-Vinylen- oder eine Acetylengruppe, eine CR²R³-Gruppe,
wobei
R² Wasserstoff. ein C₁-C₄-Alkylrest oder Phenyl,
R³ ein C₁-C₄-Mkylrest, Benzyl, Phenyl. Hydroxy oder eine Gruppe NR⁴R⁵. worin
R⁴ Wasserstoff, Benzyl, Phenyl oder ein C₁-C₄-Alkylrest und
R⁵ Wasserstoff oder ein C₁-C₄-Alkylrest sein kann, eine Gruppe Y-Z-Y,
wobei
Y Schwefel oder Sauerstoff,
Z eine Alkylkette (CH₂)ₙ und n 1-5 sein kann, bedeutet, und
- W: ein Halogenatom; eine Cyano- oder Rhodanogruppe, Aminocarbonyl; ein Methyl, Isopropyl oder tert-Butylrest; ein C₃-C₈-Cycloalkylrest, der unsubstituiert oder durch Phenyl oder C₁-C₄-Alkyl substituiert sein kann; ein Cyclohexenyl- oder Cyclopentenylrest, ein Phenylring, der substituiert sein kann durch einen oder eine beliebige Kombinationen der folgenden Substituenten: C₁-C₄-Alkyl, C₁-C₄-Alkoxy, C₁-C₄-Alkylthio, C₁-C₄-Alkylsulfinyl, C₁-C₄-Alkylsulfonyl, Trifluormethyl, Nitro, Amino, Hydroxy, Cyano, Mercapto, Sulfonamino, Acetylamino, Carboxy, Phenoxy, Benzyloxy, Phenyl, Benzoyl, Carboxy-C₁-C₄-alkyl, Methylendioxy. Ethylendioxy, Fluor, Chlor, Brom, Iod, Carboxymethoxy, Carboxyethoxy, Acetoxy, Acetyl, Propionyl, eine Gruppe NR⁶R⁷, wobei R⁶ Wasserstoff, C₁-C₄-Alkyl, Benzyl und R⁷ Wasserstoff, C₁-C₄-Alkyl, Benzyl. Phenyl oder Benzoyl ist, wobei die jeweiligen aromatischen Ringe unsubstituiert oder ein- oder mehrfach durch Halogen, Hydroxy oder C₁-C₄-Alkoxy substituiert sein können, desweiteren ein α- oder β-Naphthylring, der durch Methyl. Hydroxy, Methoxy. Carboxy. Methoxycarbonyl, Ethoxycarbonyl, Cyano. Acetyl. Chlor oder Brom substituiert sein kann oder ein Tetrahydronaphthylrest.
bedeuten,
sowie deren physiologisch verträgliche Salze oder Ester und Substanzen. Falls durch die Substitution der Alkylenkette in I mit den beschriebenen Resten chirale Verbindungen entstehen, sind sowohl die Substanzen mit R- als auch mit S-Konfiguration Gegenstand der Erfindung. Unter C₁-C₄-Alkyl-Substituenten werden geradkettige oder verzweigte Alkylreste verstanden.

Verbindungen der Formel I weisen wertvolle pharmakologische Eigenschaften auf Sie bewirken die Normalisierung erhöhter Glucosespiegel ohne begleitendes Hypoglykämierisiko und eignen sich daher hervorragend zur Therapie des Diabetes mellitus.

Bisherige Wirkprinzipien oraler Antidiabetika wie der allgemein angewendeten Sulfonylharnstoffe beruhen auf einer erhöhten Freisetzung von Insulin aus den β-Zellen des Pankreas, ein Mechanismus, der langfristig zu einer totalen Erschöpfung der Insulineigenproduktion des Diabetikers führt. Moderne Betrachtungen der Pathobiochemie des Altersdiabetes stellen daher die Notwendigkeit der Therapie der in diesem Fall vorhandenen peripheren Insulinresistenz in den Vordergrund.

Verbindungen der Formel I bewirken die Verbesserung der Glucoseverwertung z. B. im Muskel, durch Erhöhung der Insulinsensitivität bauen sie Hyperinsulinämie ab und entsprechen damit genau dem geforderten Therapiekonzept.

Diabetiker leiden häufig unter einer generellen Entgleisung der gesamten Stoffwechsellage, charakterisiert durch Hyperlipidämie. Cholesterinerhöhung, Hypertonie, Adipositas und Hyperinsulinämie, ein Krankheitsbild, welches als metabolisches Syndrom oder auch Syndrom X bezeichnet wird und Spätkomplikationen größten Ausmaßes nach sich zieht. Verbindungen der allgemeinen Formel I bewirken neben dem Abbau des Hyperinsulinismus zusätzlich eine Senkung der Triglyceride, des Cholesterins und Fibrinogens, sie eignen sich daher hervorragend zur Behandlung des metabolischen Syndroms.

Verbindungen der allgemeinen Formel I bei denen W ein Chloratom und A-B-A' eine Alkylenkette -(CH₂)ₙ- bedeutet sind bereits ohne Angabe einer pharmakologischen Wirkung beschrieben. So ist in Doklady Akad. Nauk S.S.S.R. 127, 1027(1959) die Darstellung von 2,2,8-Trichlor-octansäureethylester (n = 6) angegeben. Izvest. Akad. Nauk S.S.S.R 1960, 1215 beschreibt die Synthese von 2,2,8-Trichlor-octansäure (n = 6), 2,2,6-Trichlorhexansäure (n = 4) und 2,2,6-Trichlor-heptansäure (n = 5).

Bekannt sind weiterhin Verbindungen der allgemeinen Formel I, bei denen W eine Methylgruppe und A-B-A' eine Alkylenkette -(CH₂)ₙ- bedeutet und die als Haupt- oder Nebenprodukte bei Chlorierungsreaktionen gefunden wurden, ohne daß bisher eine Verwendung als Arzneimittel beschrieben wurde. Ind. Eng. Chem. Res. 114, 2425(1992): 2,2-Dichlordecansäure, 2,2-Dichloroctansäure und 2,2-Dichlortetradecansäure. Bull. Soc. Chim. Belg. 97, 525(1988): 2,2-Dichlordecansäure, 2,2-Dichloroctansäure, 2,2-Dichloroctadecansäure, 2,2-Dichlordodecansäure, 2,2-Dichlorhexadecansäure und 2,2-Dichlortetra- decansäure. Eur. Pat. 167.202: 2,2-Dichloroctansäure und 2,2-Dichlornonansäure. Wear 3, 200(1960): 2,2-Dichloroctadecansäure. Eur. Pat. 87.835: 2,2-Dichloroctadecansäure. Izv. Vyssh. Uchebn. Zaved., Khim. Khim. Tekhnol. 18, 674(1975): 2,2-Dichloroctadecansäure und 2,2-Dichlornonansäure. Ger. Offen. 2.264.234: 2,2-Dichlortetradecansaure. U.S. 3.573.332: 2,2-Dichlordodecansäure. Can. J. Chem. 36, 440(1958): 2.2-Dichlordodecansäure.

Bevorzugte Verbindungen der allgemeinen Formel I sind Verbindungen,
in denen
- A: eine Alkylenkette mit 8 - 14, vorzugsweise 10 - 12 Kohlenstoffatomen.
- A': ein Valenzstrich. Vinylen oder Acetylen,
- B: Valenzstrich, eine Methylengruppe, Sauerstoff, Schwefel, Sulfoxid oder Sulfonyl und
- W: ein C₃-C₈-Cycloalkyl oder ein gegebenenfalls substituierter Phenylrest, insbesondere 4-Chlorphenyl, 4-Methylthiophenyl, 4-C₁-C₄-Alkylphenyl, 4-Methyl-Sulfonylphenyl,
bedeuten.

Die Alkylenkette A bzw. A' ist vorzugsweise geradkettig, kann aber auch verzweigt sein.

Unter Halogen ist Fluor. Chlor, Brom oder Iod zu verstehen. Die C₃-C₈-Cycloalkyl-Reste bedeuten Cyclopropyl, Cyclobutyl, Cyclopentyl, Cyclohexyl, Cycloheptyl oder Cyclooctyl.
Beispiele von physiologisch verwendbaren Salzen der Verbindungen der Formel I sind Alkalimetall-, Erdalkalimetall-, Ammonium- und Alkylammoniumsalze, wie das Na-, K-, Mg-, Ca- oder Tetramethylammoniumsalz.

Beispiele von Estern sind aliphatische Alkohole, wie C₁-C₆-Alkanole, insbesondere Methyl-, Ethyl-, Propyl-, Butyl- und Isopropylester.

Die Carbonsäurederivate der allgemeinen Formel I können in flüssiger oder fester Form oral und parenteral appliziert werden Als Injektionsmedium kommt vorzugsweise Wasser zur Anwendung, welches die bei Injektionslösungen üblichen Stabilisierungsmittel. Lösungsvermittler und/oder Puffer enthält. Derartige Zusätze sind z. B. Tartrat- oder Borat-Puffer, Ethanol, Dimethylsulfoxid, Komplexbildner (wie Ethylendiamintetraessigsäure), hochmolekulare Polymere (wie flüssiges Polyethylenoxid) zur Viskositätsregelung oder Polyethylen-Derivate von Sorbitanhydriden. Feste Trägerstoffe sind z. B. Stärke. Lactose, Mannit, Methylcellulose, Talkum, hochdisperse Kieselsäure, höhermolekulare Polymere (wie Polyethylenglykole). Für die orale Applikation geeignete Zubereitungen können gewünschtenfalls Geschmacks- und Süßstoffe enthalten.

Die verabreichte Dosis hängt vom Alter, der Gesundheit und dem Gewicht des Empfängers, dem Ausmaß der Krankheit, der Art gleichzeitiger gegebenenfalls durchgeführter weiterer Behandlungen und der Art der gewünschten Wirkung ab. Üblicherweise beträgt die tägliche Dosis der aktiven Verbindung 0.1 bis 50 mg/kg Körpergewicht. Normalerweise sind 0.5 bis 40 und vorzugsweise 1.0 bis 20 mg/kg/Tag in einer oder mehreren Anwendungen pro Tag wirksam, um die gewünschten Resultate zu erhalten.

Gegenstand der vorliegenden Anmeldung sind auch Verbindungen der Formel I,
in welcher
- A: eine Alkylenkette mit 5 - 20 Kohlenstoffatomen,
- A': ein Valenzstrich, eine Vinylen- oder Acetylengruppe oder eine Alkylenkette mit 1 - 10 Kohlenstoffatomen,
- B: Valenzstrich, Schwefel, Methylen. Sauerstoff oder die Gruppe NR^{1,}
wobei
R¹ Wasserstoff. Benzyl, Phenyl oder ein C₁-C₄-Alkylrest sein kann, eine Carbonyl-, Sulfonamid-, Sulfoxid- oder Sulfongruppe, eine E- oder Z-Vinylen- oder eine Acetylengruppe, eine CR²R³-Gruppe,
wobei
R² Wasserstoff, ein C₁-C₄-Alkylrest oder Phenyl,
R³ ein C₁-C₄-Alkylrest. Benzyl, Phenyl, Hydroxy oder eine Gruppe NR⁴R⁵,
worin
R⁴ Wasserstoff. Benzyl, Phenyl oder ein C₁-C₄-Alkylrest und
R⁵ Wasserstoff oder ein C₁-C₄-Alkylrest sein kann, eine Gruppe Y-Z-Y,
wobei
Y Schwefel oder Sauerstoff.
Z eine Alkylkette (CH₂)ₙ und n 1-5 sein kann, bedeutet,
und
- W: Brom; eine Cyano- oder Rhodanogruppe, Aminocarbonyl; ein Methyl. Isopropyl oder tert-Butylrest: ein C₃-C₈-Cycloalkylrest, der unsubstituiert oder durch Phenyl oder C₁-C₄-Alkyl substituiert sein kann; ein Cyclohexenyl- oder Cyclopentenylrest, ein Phenylring, der substituiert sein kann durch einen oder eine beliebige Kombinationen der folgenden Substituenten: C₁-C₄-Alkyl, C₁-C₄-Alkoxy, C₁-C₄-Alkylthio, C₁-C₄-Alkylsulfinyl, C₁-C₄-Alkylsulfonyl, Trifluormethyl, Nitro, Amino, Hydroxy, Cyano, Mercapto, Sulfonamino, Acetylamino, Carboxy, Phenoxy, Benzyloxy, Phenyl, Benzoyl, Carboxy-C₁-C₄-alkyl, Methylendioxy, Ethylendioxy, Fluor, Chlor, Brom, Iod, Carboxymethoxy, Carboxyethoxy, Acetoxy, Acetyl, Propionyl, eine Gruppe NR⁶R⁷, wobei R⁶ Wasserstoff, C₁-C₄-Alkyl, Benzyl und R⁷ Wasserstoff, C₁-C₄-Alkyl, Benzyl, Phenyl oder Benzoyl ist, wobei die jeweiligen aromatischen Ringe unsubstituiert oder ein- oder mehrfach durch Halogen, Hydroxy oder C₁-C₄-Alkoxy substituiert sein können, desweiteren ein α- oder β-Naphthylring, der durch Methyl, Hydroxy, Methoxy, Carboxy, Methoxycarbonyl. Ethoxycarbonyl, Cyano, Acetyl, Chlor oder Brom substituiert sein kann oder ein Tetrahydronaphthylrest,
sowie deren physiologisch verträgliche Salze oder Ester.

Die Verbindungen der allgemeinen Formel I, in der A, A', B und W die oben genannten Bedeutungen besitzen, werden hergestellt, indem man eine Halogenverbindung der allgemeinen Formel II

X―A―B―A'―W (II),

mit Dichloressigsäure oder Estern der Dichloressigsäure in Gegenwart von starken Basen umsetzt. Die Reaktion wird üblicherweise in Lösungsmitteln wie Diethylether, Tetrahydrofuran, Dimethoxyethan, Diethylenglycoldimethylether oder t-Butyldimethylether bei Temperaturen zwischen -80 °C und -20 °C durchgeführt. Bevorzugte Base ist Lithiumdiisopropylamid (LDA). Die Reinigung der Produkte erfolgt üblicherweise durch Flashchromatographie an Kieselgel und/oder Umkristallisation der Natriumsalze aus Alkoholen wie Methanol. Ethanol oder Isopropanol.

Die Verbindungen der Formel II sind literaturbekannt oder können nach an sich bekannten Verfahren hergestellt werden. So kann z. B. die Synthese der Halogenverbindungen erfolgen durch Wittig-Reaktion eines aromatischen oder aliphatischen Aldehyds W-CHO mit dem Phosphoniumsalz einer α,ω-Dihalogenverbindung, gegebenenfalls gefolgt von anschließender katalytischer Hydrierung der entstandenen Doppelbindung. Alternativ kann ein aus Aryl- oder Alkylbromid W-Br durch Magnesium in die Grignard-Verbindung überführt und unter Cuprat-Katalyse nach Schlosser (Angew. Chem. 86, 50 (1974)) an α,ω-Dihalogenverbindungen gekuppelt werden.

Halogenverbindungen der allgemeinen Formel II, in denen W einen Aryl-, Alkyl- oder Cycloalkylrest bedeutet, werden erhalten, indem die entsprechende Bromverbindung W-Br durch Magnesium in die Grignard-Verbindung überführt und unter Cuprat-Katalyse nach Schlosser (Angew. Chem. 86, 50 (1974) an eine α,ω-Dihalogenverbindung gekuppelt wird.

Verbindungen II, bei denen A' oder B einer Acetylengruppe entspricht, werden durch Umsetzung der Acetylenverbindung W-C≡C-H bzw. W-A'-C≡C-H mit α,ω-Dibromalkanen in flüssigem Ammoniak in Gegenwart von Natriumamid oder in Dioxan in Gegenwart von Butyllithium synthetisiert. Durch Hydrierung der Dreifachbindung nach bekannten Methoden, z. B. am Katalysator nach Lindlar, sind Substanzen der Formel II zugänglich, in denen B bzw. A' eine Vinylengruppe bedeutet.

Handelt es sich um Verbindungen der Formel II, in denen B ein Schwefelatom darstellt, werden diese durch Reaktion der Thiole W-SH bzw. W-A'-SH mit den in dieser Erfindung beschriebenen ω-Brom-2,2-dichlorcarbonsäureestern dargestellt. Geeignet zur Durchführung dieser Reaktion sind dipolare, aprotische Lösungsmittel, vorzugsweise Dimethylformamid, in Gegenwart von anorganischen Basen wie Natriumhydrid oder Kaliumcarbonat. Die erhaltenen Thioether können auf bekannte Art und Weise durch Oxidation mit 3-Chlor-perbenzoesäure bzw. Wasserstoffperoxid in die Sulfoxide bzw. Sulfone überführt werden.

Die Darstellung von Verbindungen der allgemeinen Formel II, in denen B Sauerstoff oder Stickstoff bedeutet, erfolgt durch Umsetzung der Alkohole, Phenole W-A'-OH bzw. Amine W-A'-NHR¹ mit α,ω-Dibromalkanen, wobei die Reaktion überlicherweise in Dimethylformamid oder Dimethylsulfoxid in Gegenwart von Basen wie Natriumhydrid, Kaliumhydroxid, Triethylamin. Kaliumcarbonat oder Pyridin bei Temperaturen von 20 - 120 °C durchgeführt wird.

Im Sinne der vorliegenden Erfindung kommen außer den in den Beispielen genannten Verbindungen die folgenden Verbindungen der Formel I in Frage, die auch als Salze oder Ester vorliegen können:
1. 2,2-Dichlor-14-(3,5-di-tert-butyl-4-hydroxy-phenyl)-tetradecansäure
2. 2,2-Dichlor-14-(3,5-dimethyl-4-hydroxy-phenyl)-tetradecansäure
3. 2,2-Dichlor-14-(3-trifluormethyl-phenyl)-tetradecansäure
4. 2,2-Dichlor-14-(2-methoxy-phenyl)-tetradecansäure
5. 2,2-Dichlor-14-(2-chlor-phenyl)-tetradecansäure
6. 14-(4-Carboxy-phenyl)-2,2-dichlor-tetradecansäure
7. 12-(4-Carboxymethoxy-phenyl)-2,2-dichlordodecansäure
8. 2,2-Dichlor- 14-cyclohex-2-enyl-tetradecansäure
9. 2,2-Dichlor-14-cyclopentyl-tetradecansäure
10. cis-14-(4-tert.-Butyl-cyclohexyl)-2.2-dichlor-tetradecansäure
11. 2,2-Dichlor-12-(5,6,7,8-tetrahydro-napht-1-yl)-dodecansäure
12. 2,2-Dichlor-14-(4-cyano-phenyl )-tetradecansäure
13. 12-Biphen-4-yl-2,2-dichlor-dodecansäure
14. 10-(4-Benzyloxy-phenyl)-2,2-dichlor-decansäure
15. 2,2-Dichlor-12-(4-methylphenylsulfonylamino)-dodecansäure
16. 2,2-Dichlor-12-(4-phenoxy-phenyl)-dodecansäure
17. 14-(4-Acetylamino-phenyl)-2,2-dichlor-tetradecansäure
18. 10-(4-Benzyl-phenyl)-2,2-dichlor-decansäure
19. 2,2-Dichlor-17,17-dimethyl-octadecansäure
20. 2,2-Dichlor-14-(4-methyl-phenyl)-14-oxo-tetradecansäure
21. 2,2-Dichlor-14-(4-fluor-phenyl)-tetradecansäure
22. 2.2-Dichlor-12-(4-methylsulfonylphenyl)-dodecansäure
23. 12-(4-tert.-Butylphenyl)-2.2-dichlor-dodecansäure
24. 12-(4-tert.-Butylphenoxy)-2.2-dichlor-dodecansäure
25. 2.2-Dichlor-15-phenyl-pentadecansäure
26. 2,2-Dichlor-16-phenyl-hexadecansäure
27. 2,2-Dichlor-13-phenyl-tridecansäure
28. 2,2-Dichlor-14-cyclohexyl-tetradecansäure
29. 2,2-Dichlor-14-(4-methoxy-phenyl)-14-oxo-tetradecansäure

### Ausführungsbeispiele

### Beispiel 1:

### 12-Brom-2.2-dichlor-dodecansäure (1)

Zu einer unter Stickstoffatmosphäre aus 11.2 g (110 mmol) Diisopropylamin und 66.0 ml (105 mmol) Butyllithium (1.6 M in Hexan) bei 0 °C in 150 ml Tetrahydrofuran hergestellten Lösung von Lithiumdiisopropylamid tropfte man bei -70 °C eine Lösung von 6.41 g (49.7 mmol) Dichloressigsäure in 20 ml THF innerhalb von 30 Min. zu. Man ließ noch 30 Min, bei -70 °C rühren und versetzte die klare gelbe Lösung mit 15.0 g (50.0 mmol) 1,10-Dibromdecan gelöst in 30 ml THF und hilt die Temperatur für 6 h zwischen -50 °C und -35 °C. Der anfänglich gebildete Konzentrationsniederschlag löst sich dabei wieder auf Zur Aufarbeitung wurde mit 200 ml 3 N HCl versetzt und mit 200 ml Essigester extrahiert. Man wusch die organische Phase mit 3 N HCl und gesättigter NaCl-Lösune nach und extrahierte die wässrige Phase nochmals mit 200 ml Essigester. Die vereinigten organischen Phasen wurden über Natriumsulfat getrocknet und das Lösungsmittel abdestilliert. Flashchromatographie des Rückstandes an Kieselgel (Laufmittel: Petrolether/Essigester 4:1. 1% Eisessig) ergab 8.3 g (48%) 1, Schmp. 49-51 °C (Isohexan).

### Beispiel 2:

### 14-Brom-2,2-dichlor-tetradecansäure (2)

Analog zu Beispiel 1 aus 50.0 g (152 mmol) 1,12-Dibromdodecan und 39.3 g (305 mmol) Dichloressigsäure. Ausbeute 11.9 g (21%), Schmp. 59-60 °C.

### Beispiel 3:

### 14-Brom-2,2-dichlor-tetradecansäureethylester (3)

Zu einer Lösung von 3.30 g (8.77 mmol) 2 in 40 ml Dichlormethan gab man 1 Tropfen Dimethylformamid und 1.34 g (10.5 mmol) Oxalylchlorid. Nach 30 Min. wurde überschüssiges Oxalylchlorid im Stickstoffstrom entfernt. Anschließend tropfte man ein Gemisch aus 0.97 g (21.1 mmol) Ethanol und 2,13 g (21.1 mmol) Triethylamin bei 0 °C zu. Man ließ auf Raumtemp, kommen und rührte 30 Min. nach. Nach Zersetzen mit 60 ml Wasser wurde mit Methylenchlorid extrahiert, mit 0.5 N HCl und Wasser gewaschen, über Natriumsulfat getrocknet und das Lösungsmittel entfernt. 3.38 g (95%) farbloses Öl.

### Beispiel 4:

### 16-Brom-2,2-dichlor-hexadecansäure (4)

Analog zu Beispiel 1 aus 2.0 g (5.6 mmol) 1,14-Dibromtetradecan und 2.3 g (22.5 mmol) Dichloressigsäure. Ausbeute 0.58 g (23%). Schmp. 61-63 °C.

### Beispiel 5:

### 7-Brom-2,2-dichlor-heptansäure (5)

Unter Rühren in Stickstoffatmosphäre wurden 24.3 g (33.6 ml, 0.240 mol) Diisopropylamin in 100 ml THF gelöst und bei - 50 °C 100 ml (0.240 mol) einer 2.40 M Lösung von Butyllithium in Hexan zugetropft. Man ließ 10 Min. auf -10 °C kommen, tropfte dann bei -75 °C eine Lösung von 15.5 g (0.120 mol) Dichloressigsäure in 20 ml THF zu, rührte 25 Min. bei -75 °C und gab anschließend 93.5 g (55.0 ml, 0.41 mol) 1,5-Dibrompentan in 50 ml THF so zu, daß die Temperatur auf -40 °C anstieg. Nach 2.5 h bei -40 °C wurde mit 10 ml 6 N HCl hydrolysiert und der entstandene Niederschlag mit 20 ml Wasser aufgelöst. Man wusch die organische Phase zweimal mit wenig Wasser, trocknete über Magnesiumsulfat und entfernte das Lösungsm. im Vakuum. Flashchromatographie des Rückstandes an Kieselgel (Laufmittel: Essigester/Heptan 1:10) ergab 19.5 g (59%) 7-Brom-2,2-dichlorheptansäure 5 als farbloses Öl.

### Beispiel 6:

### 7-Brom-2,2-dichlor-heptansäureethylester (6)

Man löste 19.5 g 5 in 300 ml Ethanol, sättigte bei 0 °C mit Chlorwasserstoffgas und rührte noch 5 h bei 0 °C. Nach Entfernung der Hauptmenge an Ethanol i. Vak. wurde der Rückstand in Ether aufgenommen, mit Wasser gewaschen, über Magnesiumsulfat getrocknet und das Lösungsmittel entfernt. Man erhielt 21.2 g (98%) 7-Brom-2,2-dichlorheptansäureethylester 6 als farbloses Öl.

### Beispiel 7:

### 8-Brom-2,2-dichlor-octansäure (7)

Analog zu Beispiel 5 aus 12.7 g (52.0 mmol) 1,6-Dibromhexan und 2.2 g (17.0 mmol) Dichloressigsäure. Ausbeute 7.64 (50%), farbloses Öl.

### Beispiel 8:

### 2,2-Dichlor-12-cyano-dodecansäure (8)

Zu einer Suspension von 393 mg (9.82 mmol) Natriumhydrid (60% in Weißöl) in 30 ml DMSO tropfte man zügig eine Lösung von 3.42 g (9.82 mmol) 1 in 5 ml DMSO. Nach beendeter Wasserstoffentwicklung gab man 1.47 g (30.0 mmol) Natriumcyanid (getrocknet bei 120 °C im Hochvakuum) zu und erhitzte 45 Min. aus 50-60 °C. Nach Abkühlung wurde mit 200 ml Essigester versetzt und mit einer Lösung von 10 g Eisen(III)chlorid in 3 N HCl angesäuert. Man wusch zweimal mit ges. NaCl-Lsg. und extrahierte die wässrige Phase mit Essigester. Die vereinigten organischen Phasen wurden über Natriumsulfat getrocknet. Nach Entfernung des Lösungsmittels und Flashchromatographie an Kieselgel (Laufmittel: Petrolether/Essigester 4:1. 1% Eisessig) wurden 2.61 g (90%) 8 als hellgelbes Öl erhalten.

### Beispiel 9:

### 2,2-Dichlor-12-phenoxy-dodecansäure (9)

### 1-Brom-10-phenoxy-decan (61):

Zu einer aus 1.20 g (30.0 mmol) NaH (60% in Weißöl) und 30 ml Ethanol hergestellten Natriumethanolatlsg, gab man 2.90 g (30.8 mmol) Phenol und 9.00 g (30.0 mmol) 1,10-Dibromdecan. Die anfangs klare, schwach gelbe Lösung erhitzte man zum Rückfluß. Bereits nach 30 Min. begann sich ein Niederschlag zu bilden. Nach 6 h ließ man abkühlen, gab 300 ml Essigester zu und wusch dreimal mit 200 ml ges. Kochsalzlsg.. Nach Trocknung über Natriumsulfat und Einengen am Rotationsverdampfer wurde der Rückstand in Ethanol gelöst und 24 h im Kühlschrank aufbewahrt. Man saugte das ausgefallene Produkt ab und wusch mit wenig kaltem Ethanol nach. 6.00 g (64%) 61, Schmp. 62-64 °C.

Zu einer analog Beispiel 1 aus 7.60 g (75.0 mmol) Diisopropylamin, 46 ml (74 mmol) Butyllithium (1.6 M in Hexan) und 4.81 g (37.2 mmol) Dichloressig- säure in 80 ml THF hergestellten Enolatlsg. gab man bei -78 °C eine Lösung von 5.84 g (18.6 mmol) 61 und ließ im Kühlbad langsam auftauen. Nach Erreichen von -30 °C kühlte man nochmals auf -50 °C ab und ließ auf -20 °C kommen. Nach Zugabe von 50 ml 3 N HCl und 200 ml Essigester wurde zweimal mit je 150 ml 3 N HCl und zweimal mit ges. NaCl-Lsg. gewaschen. Nach Trocknung über Natriumsulfat und Entfernung des Lösungsmittels am Rotationsverdampfer reinigte man durch Flashchromatographie an Kieselgel (Laufmittel: Petrolether/Isopropanol 96:4, 0.5% Eisessig). 4.25 g (63%) schwach gelbes, bald zu einer wachsartigen Masse erstarrendes Öl.

### Beispiel 10:

### 2,2-Dichlor-12-(4-methyl-phenoxy)-dodecansäure (10)

### 1-Brom-10-(4-methyl-phenoxy)-decan (62):

Zu der aus 6.5 g (60 mmol) NaH (60% in Weißöl) in 60 ml Ethanol hergestellten Phenolatlsg, gab man 18 g (60 mmol) 1,10-Dibromdecan und erhitzte 6 h unter Rückfluß. Es schied sich ein farbloser Niederschlag ab. Nach Zugabe von 200 ml 3 N HCl und 200 ml Essigester wusch man zweimal mit ges. NaCl-Lsg., trocknete über Natrium-sulfat und dampfte das Lösungsm. i. Vak. ab. Aus dem Rohprodukt fiel nach Zugabe von Toluol der Diphenylether aus. Das Filtrat wurde destilliert und die Fraktion 150-160 °C(1.3 mbar) aus Essigester umkristallisiert. Ausbeute 9.75 g 62.

Das in Analogie zu Beispiel 9 aus 5.15 g (40 mmol) Dichloressigsäure und 9.5 g (29 mmol) 62 erhaltene Rohprodukt wurde durch Flashchromatographie (Kieselgel, Laufrnittel: Essigester/Petrolether 9:1,1% Eisessig) von polaren Verunreinigungen befreit. Das erhaltene Öl nahm man in Petrolether auf und fällte das Natriumsalz mit ges. Natriumhydrogencarbonatlsg, aus. Nach Filtration und Umkristallisation aus Essigester, wurde mit 3 N HCl die Säure wieder freigesetzt, mit Essigester extrahiert, über Natriumsulfat getrocknet und das Lösungsmittel abgedampft. Umkristallisation der freien Säure aus Petrolether ergab 2.4 g (22%) 10, Schmp. 67-68 °C.

### Beispiel 11:

### 2,2-Dichlor-12-(4-chlor-phenoxy)-dodecansäure (11)

### 1-Brom-10-(4-chlor-phenoxy)-decan (63):

Analog zur Darstellung von 62 erhielt man aus 7.7 g (60 mmol) 4-Chlorphenol und 18 g (60 mmol) 1,10-Dibromdecan 13.6 g (65%) 63. Entsprechend dem Beispiel 9 wurden 13.3 g (38.0 mmol) 63 mit Dichloressigsäure umgesetzt. Man erhielt 5.9 g (50%) 11, Schmp. 63-64 °C.

### Beispiel 12:

### 2,2-Dichlor-12-(4-methoxy-phenoxy)-dodecansäure (12)

### 1-Brom-10-(4-methoxy-phenoxy)-decan (64) :

Analog zur Darstellung von 62 erhielt man durch Umsetzung von 7.5 g (60 mmol) Hydrochinonmonomethylether und 18 g (60 mmol) 1,10-Dibrom- decan 8.8 g (43%) 64, Schmp. 64-66 °C

Eine aus 80.0 mmol Lithiumdiisopropylamid und 5.15 g (400 mmol) Dichloressigsäure in 50 ml THF hergestellte Enolatlsg, wurde innerhalb von 1 h bei 0 - 10 °C zu einer Lösung von 7.0 g (20 mmol) 64 in 20 ml THF getropft. Nach Rühren für 1 h hydrolysierte man mit 3 N HCI, versetzte mit 200 ml Essigester, wusch zweimal mit 3 N HCl und einmal mit ges. NaCl-Lsg. und engte die organische Phase im Vakuum ein. Der ölige Rückstand wurde in Petrolether aufgenommen und mit soviel ges. NaHCO₃-Lsg. versetzt bis keine CO₂-Entwicklung mehr zu beobachten war. Nach 30 Min. wurde der gebildete Niederschlag abgesaugt und aus Essigester umkristallisiert. Das farblose Salz nahm man in Essigester auf, versetzte mit 3 N HCI und wusch die organische Phase mit ges. Kochsalzlösung. Das nach Trocknung über Natriumsulfat und Eindampfen erhaltene Öl wurde aus Petrolether kristallisiert. 1.6 g (20%) 12 als farblose Plättchen, Schmp. 68-69 °C.

### Beispiel 13:

### 2,2-Dichlor-12-phenyl-dodec-11-ensäure (13)

### 9-Bromnonyltriphenylphosphoniumbromid (65):

103 g (0.36 mol) 1,9-Dibromnonan werden bei 120 °C gerührt und innerhalb von 8 h eine Lösung von 11.8 g (0.045 mol) Triphenylphosphin in 120 ml Toluol zu. Nach weiteren 10 h bei 120 °C ließ man abkühlen, dekantierte die überstehende Lösung ab und rührte den zähen Rückstand zweimal mit Isohexan bei 60 °C aus. Nach Trocknung am Rotationsverdampfer im Stickstoffstrom erhielt man 22.4 g (91%) 65 als fast farbloses Harz.

### 10-Brom-1-phenyl-1-decen (66):

2.13 g (3.8 mmol) 65 wurden in 200 ml THF gelöst und unter Stickstoffatmosphäre auf-78 °C gekühlt. Man tropfte 1.53 ml (3.6 mmol) Butyllithium (2.45 N in Hexan) zu, wobei die typische orangerote Ylidfarbe entsteht. Man rührte noch 30 Min. bei -78 °C und versetzte auf einmal mit 0.40 ml (4.0 mmol) frisch destilliertem Benzaldehyd, wonach sich die Lösung entfärbte. Innerhalb von 30 Min. ließ man die Temperatur auf 0 °C ansteigen und versetzte mit 5 ml gesättigter Ammoniumchloridlösung. Nach Zugabe von einigen Tropfen 2 N HCl wurde die organische Phase abgetrennt, die wässrige Phase einmal mit Ether extrahiert und die vereinigten organischen Phasen zweimal mit Wasser gewaschen. Nach Trocknung über Magnesiumsulfat und Entfernung des Lösungsmittels wurde durch Flashchromatographie an Kieselgel (Laufmittel: Heptan) gereinigt. 0.86 g (73%) 66, farbloses Öl.

Analog zu Beispiel 9 wurden 4.52 g (15.3 mmol) 66 mit 4.34 g (33.7 mmol) Dichloressigsäure zur Reaktion gebracht. Der Ansatz wurde bei -40 °C mit 6 N HCl hydrolysiert und der entstandene Konzentrationsniederschlag durch Zugabe von einigen ml Wasser aufgelöst. Man trennte die organische Phase ab, wusch mit Wasser, trocknete über Magnesiumsulfat und dampfte das Lösungsm. i. Vak. ab.

Nach Flashchromatographie an Kieselgel (Laufmittel: Heptan -> Heptan/Essigester 10:1) wurden 2.32 g (45%) 13, Schmp. 50-52 °C. erhalten.

### Beispiel 14:

### 2,2-Dichlor-12-phenyl-dodecansäure (14)

1.09 g (3.18 mmol) 13 wurden in 300 ml THF gelöst und nach Zugabe von 200 mg 10%/Pd/BaSO4 bei einem Wasserstoffüberdruck von 42 mbar 40 Min. bei -40 °C hydriert. Man saugte den Katalysator ab und erhielt nach Eindampfen der verbleibenden Lösung 0.95 g (90%) 14, farbloses Öl. 100 mg (0.29 mmol) 14 wurden in 1 ml Ethanol gelöst, im Eisbad abgekühlt und mit einer Lösung von 12 mg (0.29 mmol) Natriumhydroxid in 1 ml Ethanol versetzt. Durch Zugabe von Ether wurde das Natriumsalz ausgefällt und 12 h im Kühlschrank stehengelassen. Man saugte den Niederschlag ab, wusch mit kaltem Ether und trocknete i. Vak. 100 mg (94%) Na-Salz von 14, Schmp. 157-159 °C.

### Beispiel 15:

### 2,2-Dichlor-12-cyclohexyl-dodecansäure (15)

### 1-Brom-10-cyclohexyl-decan (67):

Zu einer Lösung von 18.0 g (60.0 mmol) 1,10-Dibromdecan in 20 ml THF gab man 10 ml (1 mmol) einer aus 1.344 g (10.0 mmol) CuCl₂ und 0.848 g (20.0 mmol) wasserfreiem Lithiumchlorid und 100 ml THF hergestellten orange-roten Lösung von Li₂CuCl₄. Anschließend wurde innerhalb von 1 h bei 0 °C eine aus 2.10 g Magnesium und 11.7 g (72.0 mmol) Cyclohexylbromid gebildete Grignard-Lösung zugetropft. Man ließ auftauen, wobei sich der Ansatz dunkel verfärbte und ein Niederschlag ausfiel. Nach 20 h Rühren gab man 50 ml ges. Ammoniumchloridlsg. und 100 ml Essigester zu, trennte die Phasen, wusch zweimal mit ges. NaCl-Lsg., trocknete die organische Phase über Natriumsulfat, entfernte das Lösungsm. am Rotationsverdampfer und fraktionierte den Rückstand durch Vakuumdestillation. 9.62 g (53%) 67, Kp. 103 - 105 °C/0.7 mbar als farblose Flüssigkeit.

Analog zu Beispiel 9 wurden aus 9.10 g (30.0 mmol) 67 und 4.64 g (36.0 mmol) Dichloressigsäure nach Flashchromatographie (Laufmittel: Petrolether/Essigester 7:3 1% Eisessig) 6.5 g farbloses Öl erhalten. Tieftemperaturkristallisation aus Toluol lieferte 4.88 g (46%) 15 vom Schmp. 67-68 °C

### Beispiel 16:

### 2,2-Dichlor-14-phenyl-tetradecansäure (16)

### 1-Brom-12-phenyl-dodecan (68):

Analog zur Darstellung von 67 (Beispiel 15) erhielt man aus 19.7 g (60.0 mmol) 1,12-Dibromdodecan, 11.31 g (72.0 mmol) Brombenzol, 2.10 g Magnesium und 10 ml (1 mmol) Li₂CuCl₄ (0.1 M in THF) 12.2 g (61%) 68 als farblose Flüssigkeit mit Kp. 130-140 °C/0.7 mbar. Zu einer - wie in Beispiel 1 - aus 7.27 g (72.0 mmol) Disopropylamin, 29 ml (72.0 mmol) Butyllithium (2.5 M in Hexan) und 4.64 g (30.0 mmol) Dichloressigsäure in THF hergestellten Enolatlsg, gab man bei -78 °C eine Lösung von 9.94 g (30.0 mmol) 68 und ließ im Kühlbad langsam auftauen. Nach Erreichen von -30 °C kühlte man nochmals auf -50 °C ab und ließ auf -20 °C kommen. Nach Zugabe von 50 ml 3 N HCl und 200 ml Essigester wurde zweimal mit je 150 ml 3 N HCI und zweimal mit ges. NaCl- Lösung gewaschen. Nach Trocknung über Natriumsulfat und Entfernung des Lösungsmittels am Rotationsverdampfer reinigte man durch Flashfiltration an Kieselgel (Laufmittel: Petrolether/Essigester 7:3 1% Eisessig). Die erhaltene Lösung wurde mit gesättigter Natriumhydrogencarbonatlsg. versetzt, das ausgefallene Natriumsalz abgesaugt, mit Petrolether gewaschen und zweimal aus Essigester umkristallisiert. Man erhielt 6.72 g (56%) des farblosen Natriumsalzes von 16, Schmp. 171 °C (Zers.).

### Beispiel 17:

### 2,2-Dichlor-10-phenyl-decansäure (17)

### 1-Brom-8-phenyl-octan (69):

Analog zur Darstellung von 67 (Beispiel 15) erhielt man aus 16.3 g(60.0 mmol) 1,8-Dibromoctan 11.31 g (72.0 mmol) Brombenzol. 2.10 g Magnesium und 10 ml (1 mmol) Li₂CuCl₄ (0.1 M in THF) 12.2 g (61%) 69 als farblose Flüssigkeit mit Kp. 110-120 °C/0.7 mbar.

Zu einer - wie in Beispiel 1 - aus 7.27 g (72.0 mmol) Diisopropylamin, 29 ml (72.0 mmol) Butyllithium (2.5 M in Hexan) und 4.64 g (30.0 mmol) Dichloressigsäure in THF hergestellten Enolatlsg. gab man bei -78 °C eine Lösung von 9.94 g (30.0 mmol) 69 und ließ im Kühlbad langsam auftauen. Nach Erreichen von -30 °C kühlte man nochmals auf -50 °C ab und ließ auf -20 °C kommen. Nach Zugabe von 50 ml 3 N HCI und 200 ml Essigester wurde zweimal mit je 150 ml 3 N HCl und zweimal mit ges. NaCl-Lsg. gewaschen. Nach Trocknung über Natriumsulfat und Entfernung des Lösungsmittels am Rotationsverdampfer reinigte man durch Flashfiltration an Kieselgel (Laufmittel: Petrolether/Essigester 7:3/1% Eisessig). Die erhaltene Lösung wurde mit gesättigter Natriumhydrogencarbonatlsg, versetzt, das ausgefallene Natriumsalz abgesaugt, mit Petrolether gewaschen und zweimal aus Essigester umkristallisiert. Man erhielt 3.5 g (35%) des farblosen Natriumsalzes von 17, Schmp. 154-156 °C.

### Beispiel 18:

### 2,2-Dichlor-7-(4-chlorphenyl)-heptansäure (18)

### 5-(4-Chlorphenyl)-pentylbromid (70):

Analog zur Darstellung von 67 (Beispiel 15) erhielt man aus 13.8 g (60.0 mmol) 1,5-Dibrompentan. 13.8 g (72.0 mmol) 4-Brom-1-chlorbenzol, 1.95 g (80 mmol) Magnesium und 10 ml (1 mmol) Li₂CuCl₄ (0.1 M in THF) 15.7 g (53%) 70 als farblose Flüssigkeit mit Kp. 115-117 °C/0.05 mbar.

Analog Beispiel 17 wurden aus 5.00 g (19.1 mmol) und 9.81 g (76.4 mmol) Dichloressigsäure nach Flashchromatographie (Petrolether/Essigester 10:1) 4.7 g (79%) 18 als farbloses Öl erhalten. Analog zu Beispiel 17 wurde das Natriumsalz von 18 hergestellt. 4.7g (74%), Schmp. 158-162 °C

### Beispiel 19:

### 2,2-Dichlor-12-(4-methylphenyl)-dodecansäure (19)

### 1-Brom-10-(4-methylphenyl)-decan (71):

Analog zur Darstellung von 67 (Beispiel 15) erhielt man aus 18.0 g (60.0 mmol) 1,10-Dibromdecan, 12.3 g (72.0 mmol) 4-Bromtoluol 2.10 g (86.0 mmol) Magnesium und 10 ml (1 mmol) Li₂CuCl₄ (0.1 M in THF) 11.0 g (57%) 71 als farblose Flüssigkeit mit Kp. 105-120 °C/0.7 mbar.

Zu einer - wie in Beispiel 1 - aus 7.27 g (72.0 mmol) Diisopropylamin, 29 ml (72.0 mmol) Butyllithium (2.5 M in Hexan) und 4.64 g (30.0 mmol) Dichlor- essigsäure in THF hergestellten Enolatlsg, gab man bei -78 °C eine Lösung von 9.94 g (30.0 mmol) 71 und ließ im Kühlbad langsam auftauen. Nach Erreichen von -30 °C kühlte man nochmals auf -50 °C ab und ließ auf -20 °C kommen. Nach Zugabe von 50 ml 3 N HCl und 200 ml Essigester wurde zweimal mit je 150 ml 3 N HCl und zweimal mit ges. NaCl-Lsg. gewaschen. Nach Trocknung über Natriumsulfat und Entfernung des Lösungsmittels am Rotationsverdampfer reinigte man durch Flashfiltration an Kieselgel (Laufmittel: Petrolether/Essigester 7:3/1% Eisessig). Die erhaltene Lösung wurde mit gesättigter Natriumhydrogencarbonatlsg, versetzt, das ausgefallene Natriumsalz abgesaugt, mit Petrolether gewaschen und aus Essigester umkristallisiert. Man erhielt 5.87 g (52%) des farblosen Natriumsalzes. Durch Aufschlämmen des Salzes in Essigester und Ansäuern mit 3 N HCl setzte man die Säure 19 frei. Nach Trocknung der organischen Phase über Natriumsulfat. Abdampfen des Lösungsmittels im Vakuum und Kristallisation aus Petrolether erhielt man 4.5 g (40%) 19, Schmp. 58-59 °C.

### Beispiel 20:

### 2,2-Dichlor-12-(4-methoxyphenyl)-dodecansäure (20)

### 1-Brom-10-(4-methoxyphenyl)-decan (72):

Analog zur Darstellung von 67 (Beispiel 15) erhielt man aus 60.0 g (0.200 mmol) 1,10-Dibromdecan, 28.0 g (0.15 mol) 4-Bromanisol. 4.8 g (0.20 mol) Magnesium und 20 ml (1 mmol) Li₂CuCl₄ (0.1 M in THF) 11.4 g (23%) 72 als farblose Flüssigkeit mit Kp. 178-190 °C/0.7 mbar. Nach der gleichen Methode wie in Beispiel 19 erhielt man aus 11.4 g (34.8 mmol) 72 und 5.15 g (40.0 mmol) Dichloressigsäure insgesamt 2.6 g (20%) farblose Verbindung 20, Schmp. 48-49 °C

### Beispiel 21:

### 2,2-Dichlor-12-(4-chlorphenyl)-dodecansäure (21)

### 1-Brom-10-(4-chlorphenyl)-decan (73):

Zu einer Lösung von 40.0 g (130 mmol) 1,10-Dibromdecan in 110 ml Tetrahydrofuran gab man 20 ml (2.0 mmol) einer Li₂CuCl₄-Lsg. (0.1 M in THF) und tropfte bei Raumtemp innerhalb von 4 h 100 ml einer 1 M 4-Chlorphenylmagnesiumbromidlösung (Aldrich) in Ether zu. Man ließ 18 h nachrühren, hydrolysierte mit 100 ml 3 N HCl, verdünnte mit 300 ml Essigester, wusch mit je 300 ml 3 N HCl, ges. NH₄Cl-Lsg. und NaCl-Lsg., trocknete die organische Phase über Natriumsulfat und engte am Rotationsverdampfer ein. Der Rückstand wurde i. Vak. fraktioniert. 8.0 g (24%) 73, Kp. 170-175 °C/0.8 mbar.

Analog Beispiel 19 erhielt man aus 8.00 g (24.0 mmol) 73 und 6.45 g (50.0 mmol) Dichloressigsäure schwach gelbes 21, nach Kristallisation aus Petrolether bei -30 °C farblos, Schmp. < Raumtemp. Zur Bildung des Na-Salzes löste man die Säure in 100 ml Essigester und versetzte mit ges. NaHCO₃-Lösung, wusch die organische Phase zweimal mit ges. NaCl-Lösung und trocknete über Natriumsulfat. Man gab soviel Petrolether zu, daß eine leichte Trübung einsetzte und ließ über Nacht bei Raumtemp. stehen. Es wurden 2.4 g (30%) des Na-Salzes von 21 erhalten, farblose Plättchen, Schmp °C.

### Beispiel 22:

### 2,2-Dichlor-7-(5-phenylpentoxy)-heptansäure (22)

### 1-Brom-5-(5-phenylpentoxy)-pentan (74):

Zu einer Suspension von 610 mg (15.0 mmol) Natriumhydrid (60% in Weißöl) in 5 ml THF tropfte man 2.40 g (14.6 mmol) 5-Phenyl-1-pentanol. Nachdem die Wasserstoffentwicklung beendet war, gab man 9.6 ml (33 mmol) 1,5-Dibrompentan zu und erhitzte 6 h auf 80 °C. Nach Flashfiltration des Reaktionsgemisches über Kieselgel (Laufmittel: Petrolether) erhielt man 8.3 g einer farblosen Flüssigkeit, aus der man durch Flashchromatographie (Petrolether) 3.50 g (76%) 74 als farblose Flüssigkeit isolierte.

Analog Beispiel 15 wurden 3.03 g (9.67 mmol) 64 mit 1.93 g (15.0 mmol) Dichloressigsäure umgesetzt. Nach Flashchromatographie (Petrolether/ Essigester 9:1, 1% Eisessig) erhielt man 2.5 g, die nach Kristallisation aus Toluol bei -30 °C 1.6 g reines 22, Schmp. 83-84 °C ergaben.

### Beispiel 23:

### 2,2-Dichlor-14-phenyl-tetradec-13-in-säure (23)

### 1-Brom-12-phenyl-dodec-11-in (75):

Zu einer auf -78 °C gekühlten Lösung von 8.20 g (80.0 mmol) Phenylacetylen in 70 ml THF tropfte man 37.2 ml (84.0 mmol) Butyllithium (2.35 M in Hexan) und versetzte anschließend mit 50.42 g (168 mmol) 1,10-Dibromdecan. Man ließ auf Raumtemperatur kommen und erhitzte 12 h zum Rückfluß. Nach Zugabe von 80 ml halbges. Ammoniumchloridlsg. wurde mit Isohexan extrahiert, über Magnesiumsulfat getrocknet und eingeengt. Der Rückstand wurde durch Kugelrohrdestillation fraktioniert, 13.4 g (52%) 75, Kp. 95 °C/0.05 mbar.

Analog zu Beispiel 17 wurden 6.70 g (20.9 mmol) 75 mit 10.8 g (83.4 mmol) Dichloressigsäure umgesetzt. Man erhielt 2.3 g (30%) 23 die in das Natriumsalz von 23 umgewandelt wurden, 1.2 g, Schmp. 155-157 °C.

### Beispiel 24:

### 2,2-Dichlor-14-phenylsulfenyl-tetradecansäure (24)

### 2,2-Dichlor-14-phenylsulfenyl-tetradecansäureethylester (76):

Zur Lösung von 10,1 g (25.0 mmol) 3 in 200 ml Dimethylformamid gab man 3.46 g (25.0 mmol) Kaliumcarbonat und 2.75 g (25.0 mmol) Thiophenol. Man rührte 12 h bei Raumtemp., versetzte mit 300 ml Wasser, extrahierte mit Ether, wusch mit Wasser, trocknete über Natriumsulfat und entfernte das Lösungsm am Rotationsverdampfer. Nach Flashchromatographie des Rückstandes (Kieselgel, Heptan/Toluol 5:1) wurden 6.62 g (61%) 76 als fast farbloses Öl erhalten.

1.5 g (3.5 mmol) 76 wurden in 3.8 ml Ethanol gelöst und mit 3.8 ml 1 N KOH versetzt. Ein nach kurzer Zeit entstandener Niederschlag wurde mit 20 ml Ethanol/Wasser 1:1 in Lösung gebracht. Nach 5 h addierte man 1 ml 1 N KOH und rührte noch weitere 6 h. Nach Ansäuern mit 2 N HCl wurde mit Ether extrahiert, über Magnesiumsulfat getrocknet und das Lösungsmittel entfernt. Man erhielt 1.18 g (92%) 24, farblose Kristalle mit Schmp. 74 °C.

### Beispiel 25:

### 2,2-Dichlor-14-phenylsulfinyl-tetradecansäure (25)

### 2,2-Dichlor-14-phenylsulfinyl-tetradecansäureethylester (77):

Zur Lösung von 1.80 g (4.15 mmol) 76 in 30ml Dichlormethan gab man bei 0 bis -5 °C 0.72 g (4.15 mmol, 0.96 g 75%ige Säure) Metachlorperbenzoesäure gelöst in 15 ml Dichlormethan. Innerhalb von von 2 h ließ man auf Raumtemp. kommen und wusch die organische Phase mit Natriumhydrogencarbonatlsg, und Wasser. Nach Trocknung über Magnesiumsulfat und Entfernung des Lösungsm, wurde durch Flashchromatographie (Heptan/Essigester 2:1) gereinigt, 1.24 g (66 %), 77, farbloses Öl.

0.46g (1.02 mmol) 77 wurden mit 2.0 ml Ethanol und 2.0 ml 1 N KOH 2 h bei Raumtemp, gerührt und anschließend mit 2 N HCl angesäuert. Man extrahierte mit Ether, wusch mit Wasser, trocknete (Natriumsulfat) und erhielt nach Abdampfen des Lösungsm. 0.41 g (95%) 25 als farbloses Öl, welches nach Aufbewahrung im Kühlschrank kristallisierte, Schmp. 68 °C.

### Beispiel 26:

### 2.2-Dichlor-14-phenylsulfonyl-tetradecansäure (26)

### 2,2-Dichlor-14-phenylsulfonyl-tetradecansäureethylester (78):

Zur Lösung von 1.50 g (3.46 mmol) 76 in 15 ml Eisessig gab man 4.5 ml 30%iges Wasserstoffperoxid, rührte 48 h und goß in Eiswasser. Nach Extraktion mit Ether. Trocknung (Natriumsulfat) und Entfernung des Lösungsm. wurden 1.23 g (77%) 78 als farbloses Öl erhalten.

1.22 g (2.62 mmol) 78 wurden mit 5.2 ml Ethanol und 5.2 ml 1 N KOH versetzt und 3 h gerührt. Man kühlte auf 0 °C und säuerte mit 2 N HCl an. Der ausgefallene Niederschlag wurde abgesaugt, mit Wasser und Isohexan gewaschen und im Vakuum getrocknet. 1.12 g (97%) 26, farblose Kristalle, Schmp. 69-71 °C.

### Beispiel 27:

### 2,2-Dichlor-7-[5-(4-chlorphenyl)pentylsulfenyl]-heptansäure (27)

### 5-(4-Chlorphenyl)-1-pentanthiol (79):

Zur Lösung von 10.6 g (0.139 mol) Thiohamstoff in 40 ml Ethanol gab man 24.2 g (92.6 mmol) 5-(4-Chlorphenyl)-pentylbromid 60 in 60 ml Ethanol. Man erhitzte 5 h zum Rückfluß, ließ abkühlen, versetzte mit 50 ml konzentrierter Ammoniaklsg, und erhitzte erneut für 3 h zum Rückfluß. Nach Abkühlung wurde mit ca. 30 ml konz. HCl auf pH 1 angesäuert und zweimal mit 150 ml Ether extrahiert. Man wusch mit ges. NaCl-Lsg., trocknete über Natriumsulfat und zog das Lösungsmittel am Rotationsverdampfer ab. 19.0 g (95%) 79. Eine Mischung aus 5.00 g (16.3 mmol) 79, 2.25 g (16.3 mmol) Kaliumcarbonat, 3.50 g (16.3 mmol) 6 und 50 ml Dimethylformamid wurde 12 h bei Raum- temperatur gerührt. Man versetzte mit Wasser und extrahierte mit Ether. Die Etherphase wurde mit Wasser gewaschen, über Natriumsulfat getrocknet und eingedampft. 7.1 g gelbes Öl, das nach Flashchromatographie (Toluol/Heptan 1:2) 5.06 g (71%) des Ethylesters von 27 ergab.

Eine Mischung aus 1.50 g (3.41 mmol) des erhaltenen Esters, 6.82 ml (6.82 mmol) 1 N KOH und 7 ml Ethanol wurde 3 h bei Raumtemp. gerührt. Nach Ansäuern mit 2 N HCl und Extraktion mit Ether wusch man mit Wasser und trocknete (Na₂SO₄). Nach Entfernung des Lösungsm. verblieben 1.21 g (86%) 27 als farbloses Öl. Aus 1.13 g 27 und 110 mg Natriumhydroxid wurde das Natriumsalz hergestellt und mit Ether gewaschen. 0.75 g (64%), Schmp. 155-157 °C.

### Beispiel 28:

### 2,2-Dichlor-14-(4-isopropylphenoxy)-tetradecansäure (28)

### 1-Brom-12-(4-isopropylphenoxy)-dodecan (80):

Analog der Darstellung von 62 (Beispiel 10) erhielt man aus 8.85 g (65.0 mmol) 4-Isopropylphenol, 1.60 g (65 mmol) Natriumhydrid und 23.0 g (70.0 mmol) 1,12-Dibromdodecan nach Flashchromatographie (Essig- ester/Heptan 1:10) 10.0 g (41%) 80 vom Schmp. 51-52 °C (Ether).

Wie in Beispiel 9 wurden 4.00 g (10.0 mmol) 80 mit 5.16 g (40.0 mmol) Dichloressigsäure umgesetzt. 0.9 g (21%) 28, Schmp. 47-49 °C. Das aus 28 und Natriumhydroxid in Ethanol erhaltene Natriumsalz schmolz bei 109 °C (Zers.).

### Beispiel 29:

### 2,2-Dichlor-12-(2,6-diisopropylphenoxy)-dodecansäure (29)

### 1-Brom-10-(2,6-diisopropylphenoxy)-decan (81):

Analog zur Darstellung von 62 (Beispiel 10) erhielt man aus 11.6 g (65.0 mmol) 2,6-Diisopropylphenol, 1.60 g (65.0 mmol) Natriumhydrid und 21.0 g (70.0 mmol) 1,10-Dibromdecan nach Flashchromatographie (Essigester/Heptan 1:10) 13.95 g (54%) 81 als hellgelbes Öl.

Wie in Beispiel 9 wurden 7.95 g (20.0 mmol) 81 mit 10.3 g (80.0 mmol) Dichloressigsäure zur Reaktion gebracht. Flashchromatographie (Essigester/Heptan 1:10) ergab 4.7 g (53%) 29 als helles Öl.

### Beispiel 30:

### 2,2-Dichlor-14-[4-(4-chlorphenylcarbonylamino)phenylsulfenyl]-tetradecansäure (30)

### 2,2-Dichlor-14-[4-(4-chlorphenylcarbonylamino)phenylsulfenyl]-tetradecansäureethylester (82):

Zur Lösung von 700 mg (2.83 mmol) 4-(4-Chlorbenzoylamino)-thiophenol in 10 ml Dimethylformamid gab man 390 mg (2.83 mmol) Kaliumcarbonat und 1.14 g (2.83 mmol) 3 und rührte 50 h bei Raumtemperatur. Unter Kühlung wurde mit 20 ml Wasser versetzt, der Niederschlag abgesaugt, mit Isohexan gewaschen und im Vak. getrocknet. Die erhaltenen 1.3 g (78%) Rohprodukt wurden durch Flashchromatographie (Toluol) gereinigt. 0.82 g (50%) 82, Schmp. 130-131 °C (Dichlormethan/Isohexan).

0.68 g (1.16 mmol) 82, 2.3 ml 1 N KOH und 8 ml Ethanol wurden 2 h bei Raumtemp, gerührt. Man säuerte in der Kälte mit 2 N HCl an, verdünnte mit Wasser, extrahierte mit Ether, wusch mit Wasser, trocknete (Na₂SO₄) und zog das Lösungsm. ab. 0.61 g (98%) 30. Es wurde in 1 ml Ethanol gelöst und in der Kälte mit 44 mg NaOH in 0.5 ml Ethanol versetzt. Nach Fällung durch Zugabe von Ether, Absaugen und Waschen mit Ether wurden 0.46 g (61%) Natriumsalz von 30 mit Schmp. 167-168 °C erhalten.

### Beispiel 31:

### 2,2-Dichlor-12-(2-naphthyl)-dodecansäure (31)

### 1-Brom-10-(2-naphthyl)-decan (83):

Analog zur Darstellung von 67 (Beispiel 15) erhielt man aus 16.5 g (55.0 mmol) 1,10-Dibromdecan, 13.3 g (64.2 mmol) 2-Bromnaphthalin, 1.7 g (70 mmol) Magnesium und 10 ml (1 mmol) Li₂CuCl₄ (0.1 M in THF) nach Flashchromatographie (Essigester/Heptan 1:10) 3.7 g (20%) 83 als fahlgelbes Öl.

Wie in Beispiel 9 wurden aus 3.5 g (10.0 mmol) 83 und 5.16 g (40.0 mmol) Dichloressigsäure 3.1 g (79%) 31 erhalten, Schmp. 66-67 °C (Ether).

### Beispiel 32:

### 2.2-Dichlor-12-(4-methylsulfenylphenyl)-dodecansäure (32)

### 1-Brom-10-(4-methylsulfenylphenyl)-decan (84):

Analog zur Darstellung von 67 (Beispiel 15) erhielt man aus 16.5 g (55.0 mmol) 1,10-Dibromdecan. 13.1 g (64.2 mmol) 4-Bromthioanisol, 1.7 g (70.0 mmol) Magnesium und 10 ml (1.0 mmol) Li₂CuCl₄ (0.1 M in THF) nach Flashchromatographie (Gradientenelution, Heptan -> Heptan/Essigester 10:1) 11.2 g (59%) 84 als wachsartige Masse.

Wie in Beispiel 9 wurden aus 6.9 g (20.0 mmol) 84 und 10.32 g (80.0 mmol) Dichloressigsäure nach Flashchromatographie (Heptan/Essigester 10:1 -> Heptan/Essigester 3:1) 1.1 g (14%) 32 als farbloses Öl erhalten. Das analog zu Beispiel 30 bereitete Natriumsalz von 32 zeigt Zersetzung ab 143 °C.

### Beispiel 33:

### 2,2-Dichlor-7-[4-(4-chlorphenylcarbonylamino)phenylsulfenyl]-heptansäure (33)

### 2,2-Dichlor-7-[4-(4-chlorphenylcarbonylamino)phenylsulfenyl]-heptansäureethylester (85):

Analog zur Darstellung von 82 (Beispiel 30) wurden aus 940 mg (3.79 mmol) 4-(4-Chlorbenzoylamino)-thiophenol, 10 ml Dimethylformamid, 520 mg (3.79 mmol) Kaliumcarbonat und 1.60 g (3.79 mmol) 6 nach Flashchromatographie (Toluol) 1.14 g (63%) 85, Schmp. 136-137 °C (Essigester/Isohexan), erhalten. Durch Verseifung (Beispiel 30) von 0.41 g (0.96 mmol) 85 erhielt man 0.25 g (57%) 33, Schmp. 140-142°C.

### Beispiel 34:

### 2,2-Dichlor-8-[5-(4-chlorphenyl)pentylsulfenyl]-octansäure (34)

Analog zu Beispiel 27 wurden 3.50 g (16.3 mmol) 79 mit 5.2 g (16.3 mmol) Ethylester von 7 zur Reaktion gebracht. Nach Flashchromatographie (Heptan/ Toluol 2:1) erhielt man 5.2 g (70%) 2,2-Dichlor-8-[5-(4-chlorphenyl)-pentylsulfenyl]-octansäureethylester 86 als farbloses Öl. 2.5 g (5.5 mmol) 86, 11 ml (11 mmol) 1 N KOH und 11 ml Ethanol wurden bei Raumtemp. 2 h gerührt. Unter Kühlung im Eisbad säuerte man mit 2 N HCl auf pH 2 an, destillierte Ethanol ab, extrahierte mit Ether, wusch mit Wasser, trocknete über Na₂SO₄ und erhielt nach Entfernung des Lösungsmittels 2.24 g (96%) 34 als farbloses Öl. Aus 1.41 g (3.29 mmol) 34 in 3 ml Ethanol und 0.13 g (3.3 mmol) Natriumhydroxid in 5 ml Ethanol wurden nach Vermischen, Versetzen mit Ether, Absaugen und Trocknen 1.14 g (78%) Natriumsalz von 34 mit Schmp. 154 °C erhalten.

### Beispiel 35:

### 12-Carbamoyl-2,2-dichlor-dodecansäure (35)

200 mg (0.70 mmol) 8 wurden in 10 ml 80%ige Schwefelsäure eingetragen und 6 h auf Raumtemperatur gehalten. Man goß die erhaltene Lösung in 150 ml Eiswasser, saugte den beigefarbenen Niederschlag ab und wusch mit Petrolether nach. 180 mg (85 %) 35, Schmp. 93 - 94 °C.

### Beispiel 36:

### 2,2-Dichlor-12-(4-methylsulfinylphenyl)-dodecansäure (36)

391 mg (1.00 mmol) 32 wurden in 10 ml Dichlormethan gelöst und bei -5 bis 0 °C mit einer Lösung von 173 mg (1.00 mmol) m-Chlorperbenzoesäure in 10 ml Dichlormethan versetzt. Man ließ auf Raumtemperatur kommen, rührte noch 2 h nach und verdünnte die Mischung mit Eiswasser. Es wurde mit Dichlormethan extrahiert, über Na₂SO₄ getrocknet, mit Aktivkohle behandelt und eingedampft. Die erhaltenen 0.5 g Rohprodukt reinigte man durch Flashchromatographie (Toluol/Dioxan/Eisessig 15:12:1) und erhielt 0.20 g (50 %) 36 mit Schmp. 74 - 76 °C.

### Beispiel 37:

### 2,2-Dichlor-7-[5-(4-chlorphenyl)pentylsulfinyl]-heptansäure (37)

### 2,2-Dichlor-7-[5-(4-chlorphenyl)pentylsulfinyl]-heptansäure-ethylester (87):

1.60 g (3.64 mmol) 2,2-Dichlor-7-[5-(4-chlorphenyl)pentylsulfenyl]-heptansäureethylester (Beispiel 27) wurde in 30 ml Dichlormethan gelöst und bei -5 °C eine Lösung von 0.63 g (3.64 mmol) m-Chlorperbenzoesäure in 15 ml Dichlormethan zugetropft. Man rührte 2 h bei 0 °C, saugte die ausgefallene 3-Chlorbenzoesäure ab, wusch zweimal mit Natriumhydrogencarbonatlösung, zweimal mit Wasser, trocknete über Mageniumsulfat, konz. i. Vak. und reinigte durch Flashchromatographie (Heptan/Essigester 2:1). 1.2 g (73 %) 87 als farbloses Öl.

1.00 g (2.20 mmol) 87 wurden mit 4.4 ml (4.4 mmol) 1 N KOH und 4.4 ml Ethanol versetzt. Man rührte 4 h bei Raumtemperatur, säuerte in der Kälte auf pH 2 an. Die Säure fiel dabei als farbloser feinkristalliner Niederschlag aus, der nach 10 Min. Rühren abgesaugt und mit Isohexan/Ether 10:1 gewaschen und im Vak. getrocknet wurde. 0.86 g (92 %) 37, Schmp. 84 - 85 °C.

### Beispiel 38:

### 2,2-Dichlor-14-(4-chlorphenyl)-tetradec-8-in-säure (38)

4.5 g (12.4 mmol) 7-(4-Chlorphenyl)-hept-1-in 88 wurden in einem Gemisch aus 100 ml Dioxan und 40 ml Toluol gelöst, auf-10 °C gekühlt, mit 5.1 ml (12.5 mmol) Butyllithium (2.46 M in Hexan) und anschließend mit 9.7 g (25 mmol) 1,5-Dibrompentan versetzt. Man erhitzte 14 h auf 80 °C, anschließend 9 h auf 100 °C und ließ abkühlen. Es wurde mit 3 N HCl versetzt und mit Isohexan extrahiert. Man wusch die organischen Phase mit Wasser, trocknete über Magnesiumsulfat, engte ein und destillierte den Rückstand im Kugelrohr. 3.95 g 1-Brom-12-(4-chlorphenyl)-dodec-6-in 89, Kp. 120 °C/0.2 mbar.

Analog zu Beispiel 9 wurden 3.90 g (11.1 mmol) 1-Brom-12-(4-chlorphenyl)-dodec-6-in 89 mit 11.4 g (89 mmol) Dichloressigsäure umgesetzt, 2.00 g (45 %) 38 als farbloses Öl.

### Beispiel 39

### 2,2-Dichlor-14-(4-tert. butylphenyl)-tetradecansäure (39)

### 1-Brom-12-(4-tert, butylphenyl)-dodecan (90)

Analog zur Darstellung von 67 (Beispiel 15) erhielt man aus 18.0 g (55 mmol) 1.12- Dibromdodecan, 13.7 g (64.2 mmol) 4-tert. Butylbrombenzol, 1.7 g (70 mmol) Magnesium und 10 ml (1 mmol) Li₂CuCl₄ (0.1 M in THF) nach Flashchromatographie (Essigester/Heptan 1:10) 4.3 g (21%) 90 als fahlgelbes Öl (Siedepunkt 126 - 128°C / 0.2 mbar).

Wie in Beispiel 9 wurden aus 3.9 g (10.2 mmol) 90 und 3.87 g (30 mmol) Dichloressigsäure 1.5 g (35%) 39 erhalten. Schmp. 47 - 48°C.
Analog zu Beispiel 30 wurde das Natriumsalz erhalten. Zersetzung > 174°C.

### Beispiel 40

### 2,2-Dichlor-12-(4-tert. butylphenyl)-dodecansäure (40)

### 1-Brom-10-(4-tert. butylphenyl)-decan (91)

Analog zur Darstellung von 67 (Beispiel 15) erhielt man aus 16.5 g (55 mmol) 1.10-Dibromdodecan 13.7 g (64.2 mmol) 4-tert. Butylbrombenzol 1.7 g (70 mmol) Magnesium und 10 ml (1 mmol) Li₂CuCl₄ (0.1 M in THF) nach Flashchromatographie (Essigester/Heptan 1:10) 4.6 g (24%) 91 als fahlgelbes Öl (Siedepunkt 134°C / 0.2 mbar).

Wie in Beispiel 9 wurden aus 4,6 g (13.0 mmol) 91 und 6.7 g (52.0 mmol) Dichloressigsäure 1.1 g (22%) 40 erhalten. Schmp. 46 - 48°C. Analog zu Beispiel 30 wurde das Natriumsalz dargestellt. Zersetzung > 176°C.

### Beispiel 41

### 2,2-Dichlor-12-(4-tert. butylphenoxy)-dodecansäure (41)

### 1-Brom-10-(4-tert. butylphenoxy)-decan (92)

Analog zur Darstellung von 62 erhielt man aus 9.75 g (65.0 mmol) 4-tert. Butylphenol, 1.60 g (65 mmol) Natriumhydrid und 21.0 g (70.0 mmol) 1,10-Dibromdecan nach Destillation bei 170-175°C / 0.06 mbar 16.1 g (67%) 92 als gelbes Öl.

Wie in Beispiel 9 wurden 6.8 g (20 mmol) 92 mit 10.3 g (80 mmol) Dichloressigsäure umgesetzt. Man erhielt 2.8 g (35%) 41 vom Schmelzpunkt 56 - 57 °C. Das aus 41 und pulverisierter NaOH in Ethanol erhaltene Natriumsalz schmolz bei 178°C (Zers.)

### Beispiel 42

### 2,2-Dichlor-15-phenyl-pentadecansäure (42)

### 1-Brom-13-phenyl-tridecan (93)

Analog zur Darstellung von 67 (Beispiel 15) erhielt man aus 7.48 g (27.5 mmol) Dibromoctan, 7.27 g (32.1 mmol) 1-Brom-5-phenylpentan, 0.85 g (35 mmol) Magnesium und 5 ml (0.5 mmol) Li₂CuCl₄ (0.1 M in THF) nach Destillation am Hochvakuum 4.9 g (51%) 93 als farbloses Öl. Vom Siedepunkt 158 - 159°C / 0.15 mbar.

Wie in Beispiel 9 wurden aus 1.7 g (5 mmol) 93 und 2.58 g (20 mmol) Dichloressigsäure 1.3 g (67%) 42 erhalten. Schmp. 52 - 53°C.
Aus 0.8 g (2,1 mmol) 42 wurden mit 84 mg (2.1 mmol) NaOH-Pulver das Natriumsalz hergestellt. 0.7 g Schmp. 170°C (Zers.)

### Beispiel 43

### 2,2-Dichlor-13-phenyl-tridecansäure (43)

### 1-Brom-11-phenyl-undecan (94)

Analog der Darstellung von 67 (Beispiel 15) erhielt man aus 12.65 g (55 mmol) Dibrompentan, 15.5 g (64.2 mmol) 1-Brom-6-phenylhexan, 1.7 g (70 mmol) Magnesium und 10 ml (1 mmol) Cuprat-Lösung nach Destillation am Hochvakuum 7.5 g (44%) 94 als farbloses Öl vom Siedepunkt 150 - 152°C / 0.4 mbar.

Wie in Beispiel 9 wurden aus 6.23 g (20 mmol) 94 und 10.3 g (80 mmol) Dichloressigsäure 5.1 g (71%) 43 erhalten. Schmp. 46 - 47°C.
Das aus 43 und pulverisierter NaOH in Ethanol erhaltene Natriumsalz schmolz bei 165 °C (Zers.).

### Beispiel 44

### 2,2-Dichlor-16-phenvl-hexadecansäure (44)

### 1-Brom-14-phenyl-tetradecan (95)

Analog der Darstellung von 67 (Beispiel 15) erhielt man aus 14.96 g (55 mmol) Dibromoctan, 15.5 g (64.2 mmol) 1-Brom-6-phenylhexan, 1.7 g (70 mmol) Magnesium und 10 ml Cuprat-Lösung nach Destillation 8.7 g (45%) 95 als farbloses Öl vom Siedepunkt 168°C / 0.15 mbar.

Wie in Beispiel 9 wurden aus 7.1 g (20 mmol) 95 und 10.3 g (80 mmol) Dichloressigsäure 1.55 g (20%) 44 erhalten. Schmp. 58 - 59°C. Das aus 44 und pulverisierter NaOH in Ethanol erhaltene Natriumsalz schmolz bei 166°C (Zers.)

### Beispiel 45

### 2,2-Dichlor-14-cyclohexyl-tetradecansäure (45)

### 1-Brom-12-cyclohexyl-dodecan (96)

Analog der Darstellung von 67 (Beispiel 15) erhielt man aus 15.86 g (65 mmol) Dibromhexan, 19.8 g (80 mmol) 1-Brom-6-cyclohexyl-hexan, 2.42 g (0.1 mmol) Magnesium und 10 ml Cuprat-Lösung nach Flashchromatographie an Kieselgel (Laufmittel: Heptan, Heptan /Essigester 10:1) 10.6 g (49%) 96 als farbloses Öl.

Analog zu Beispiel 5 wurden aus 7.5 g (22.63 mmol) 96 und 8.75 g (67.89 mmol) Dichloressigsäure 1.65 g (20%) 45 erhalten mit dem Schmp.: 68-69°C. Das aus 45 und pulverisierter NaOH in Ethanol erhaltene Natriumsalz schmolz bei 146 - 148 °C.

### Beispiel 46

### 2,2-Dichlor-13-cyclohexyl-tridecansäure (46)

### 1-Brom-11-cyclohexylundecan (97)

Analog der Darstellung von 67 (Beispiel 15) erhielt man aus 17.6 g (75 mmol) 1,6-Dibromhexan, 11.66 g (50 mmol) 1-Brom-5-cyclohexylpentan, 1.46 g (60 mmol) Magnesium und 10 ml Cuprat-Lösung nach Destillation 6.81 g (43%) 97 als farbloses Öl vom Siedepunkt 106 - 110°C / 0,006 mbar.

Analog zu Beispiel 5 wurden aus 3.0 g (9.45 mmol) 97 und 3.66 g (28.36 mmol) Dichloressigsäure 2.16 g (62%) 46 vom Schmp. 50 - 51°C. Das aus 46 und pulverisierter NaOH in Ethanol hergestellte Natriumsalz schmolz bei 166 - 168°C (Zers.)

### Beispiel 47

### 2,2-Dichlor-15-cyclohexyl-pentadecansäure (47)

### 1-Brom-13-cyclohexyl-tridecan (98)

Analog der Darstellung von 67 (Beispiel 15) erhielt man aus 20.4 g (75 mmol) 1,8-Dibromoctan, 11.66 g (50 mmol) 1-Brom-5-cyclohexylpentan, 1.46 g (60 mmol) Magnesium und 10 ml Cuprat-Lösung nach Destillation 8.14 g (47%) 98 als farbloses Öl vom Siedepunkt 121 - 125°C / 0.005 mbar.

Analog zu Beispiel 5 wurden aus 4.2 g (12.24 mmol) 98 und 4.74 g (36.72 mmol) Dichloressigsäure 1.0 g (21%) 47 vom Schmp. 53 - 56°C erhalten. Das aus 47 und pulverisierter NaOH in Ethanol hergestellte Natriumsalz schmolz bei 162 - 164°C.

### Beispiel 48

### 2,2-Dichlor-16-cyclohexyl-hexadecansäure (48)

### 1-Brom-14-cyclohexyl-tetradecan (99)

Analog der Darstellung von 67 (Beispiel 15) erhielt man aus 10.87 g (38 mmol), 1,9-Dibromnonan, 5.6 g (24 mmol) 1-Brom-5-cyclohexyl-pentan, 0.73 g (30 mmol) Magnesium und 5 ml Cuprat-Lösung nach Destillation 4.5 g (53%) 99.

Analog zu Beispiel 5 wurden aus 4.4 g (12.24 mmol) 99 und 4.74 g (36.72 mmol) Dichloressigsäure 3.53 g (73%) 48 vom Schmp. 72 - 73°C erhalten. Das aus 48 und pulverisierter NaOH in Ethanol hergestellte Natriumsalz schmolz bei 156 - 158 °C.

### Beispiel 49

### 2.2-Dichlor-17-cyclohexyl-heptadecansäure (49)

### 1-Brom-15-cyclohexyl-pentadecan (100)

Analog der Darstellung von 67 (Beispiel 15) erhielt man aus 22.51 g (75 mmol) 1,10-Dibromdecan, 11.7 g (40 mmol) 1-Brom-5-cyclohexylpentan, 1.21 g (50 mmol) Magnesium und 5 ml Cuprat-Lösung nach Destillation 24.1 g (86%) 100.

Analog zu Beispiel 5 wurden aus 6.5 g (17.4 mmol) 100 und 6.73 g (52.21 mmol) Dichloressigsäure 1.84 g (25%) 49 erhalten. Schmp. 65 - 66 C. Das aus 49 und pulverisierter NaOH in Ethanol hergestellte Natriumsalz schmolz bei 152 - 155°C (Zers.)

### Beispiel 50

### 2,2-Dichlor-14-(4-chlorphenyl)-tetradecansäure (50)

### 1-Brom-6-(4-chlorphenyl)-hexan (101)

Analog der Darstellung von 67 (Beispiel 15) erhielt man aus 50 ml (330 mmol) 1,6-Dibromhexan, 30 g (160 mmol) 101, 4-Chlorbrombenzol, 3.8 g (160 mmol) Magnesium und 20 ml Cuprat-Lösung nach Destillation über Vigreux-Kolonne 20.5 g (47%) 101. Vom Siedepunkt 158 - 162°C/ 3.5 mbar.

### 1-Brom-12-(4-chlorphenyl)-dodecan (102)

Analog der Darstellung von 67 (Beispiel 15) erhielt man aus 25 ml (140 mmol) 1,6-Dibromhexan, 20 g (72 mmol) 91, 1.8 g (72 mmol) Magnesium und 20 ml Cuprat-Lösung nach Flashchromatographie an Kieselgel 18.5 g (71%) 102.

Analog zu Beispiel 5 wurden aus 10.8 g (30 mmol) 102 und 6.45 g (50 mmol) Dichloressigsäure 8.6 g (70%) 50 erhalten. Das aus 50 und pulverisierter NaOH in Ethanol hergestellte Natriumsalz schmolz bei 163 - 164 °C.

### Beispiel 51

### 2,2-Dichlor-12-(4-methylsulfonyl-phenyl)-dodecansäure (51)

4.0 g (10,2 mmol) 32 und 3.4 ml Wasserstoffperoxyd 30%ig in 10 ml Eisessig 1 Stunde auf 90°C erhitzt und in Eiswasser gegossen. Nach Extraktion mit Ether, Trocknung (Natriumsulfat) und Entfernung des Lösungsmittels wurden 3.8 g (84%) 51 vom Schmp. 167 - 168°C erhalten.

### Beispiel 52

### 2,2-Dichlor-hexadecansäure (52)

Analog zu Beispiel 5 wurden aus 5,55 g (20 mmol) 1-Tetradecylbromid (Aldrich) und 7,74 g (60 mmol) Dichloressigsäure 2,45 g (38%) 52 erhalten. Schmp. 34-37° C. Das aus 52 und pulverisierter NaOH in Ethanol hergestellte Natriumsalz schmolz bei 165 - 168°C.

### Beispiel 53

### 2,2-Dichlor-eicosansäure (53)

Analog zu Beispiel 5 wurden aus 11,1 g (33.34 mmol) 1-Octadecylbromid (Aldrich) und 12.89 g (0.1 mmol) Dichloressigsäure 10.4 g (82%) 53 erhalten. Schmp. 49 - 51°C. Das aus 53 und pulverisierter NaOH in Ethanol hergestellte Natriumsalz schmolz bei 147 - 149°C.

### Beispiel 54

### 2,2-Dichlor-12-(4-chlorphenyl-sulfenyl)-dodecansäure (54)

### 12-Brom-2,2-dichlor-dodecansäureethylester (103)

Analog zu Beispiel 3 wurden 10.0 g (28.7 mmol) 1 mit 4.19 g (33 mmol) Oxalylchlorid, 3.04 g (66 mmol) Ethanol und 6.67 g (66 mmol) Triethylamin zu 103 umgesetzt. 9.9 g (92%) farbloses Öl.

### 2,2-Dichlor-12 (4-chlorphenylsulfenyl)-dodecansäureethylester (104)

Analog zu Beispiel 30 (Darstellung von 82) wurden aus 1.85 g (12.76 mmol) 4-Chlorthiophenol, 100 ml DMF, 1.76 g (12.76 mmol) Kaliumcarbonat und 4.8 g (12.76 mmol) 103 als hellgelbes Öl 5.24 g (94%) 104 erhalten.

Durch Verseifung (Beispiel 30) von 1.65 g (3.73 mmol) 104 erhielt man 1.29 g (84%) 54. Schmp. 74 - 78°C.
Das aus 54 und pulverisierter NaOH in Ethanol hergesellte Natriumsalz schmolz bei 154-157°C.

### Beispiel 55

### 2,2-Dichlor-12 (4-chlorphenvl-sulfinyl)-dodecansäure (55)

### 2,2-Dichlor-12 (4-chlorphenyl-sulfinyl)-dodecansäureethylester (105)

Zur Lösung von 1.5 g (3.41 mmol) 104 in 30 ml Dichlormethan wurden analog Beispiel 25 (Darstellung von 77) 0.59 g (3.41 mmol) Metachlorperbenzoesäure gelöst in 15 ml Dichlormethan zugetropft. Man erhielt nach Flashchromatographie an Kieselgel 1.24 g (80%) 105. 1.24 g (2,72 mmol) 105 wurden mit 5.5 ml Ethanol und 5.5 ml 1 N KOH versetzt und 5 Stunden bei Raumtempertaur gekühlt. Man kühlte auf 0°C und säuerte mit 2 N HCl an. Der ausgefallene Niederschlag wurde abgesaugt, mit Wasser und Isohexan gewaschen und im Vakuum getrocknet 0.65 g (56%) 55.
Das aus 55 und pulverisierter NaOH in Ethanol hergestellte Natriumsalz schmolz bei 91 - 94°C.

### Beispiel 56

### 2,2-Dichlor-12 (4-chlorphenvl-sulfinyl)-dodecansäure (56)

### 2,2-Dichlor-12 (4-chlorphenyl-sulfinyl)-dodecansäureethylester (106)

Analog 78 (Beispiel 26) wurden 2.0 g (4.55 mmol) 104 mit 6 ml 30%igem Wasserstoffperoxid in 20 ml Eisessig zu 106 oxidiert. 2.08 g (99%) farbloses Öl.

Durch Verseifung (Beispiel 26) wurden aus 2 g (4.24 mmol) 106 und 8.5 ml (8.48 mmol) 1 N KOH 1.8 g (96%) 56 erhalten. Schmp. 84°C.
Das aus 56 und pulverisierter NaOH in Ethanol hergestellte Natriumsalz schmolz bei 144 - 147°C.

### Beispiel 57

### 2,2-Dichlor-14-phenyl-tetradecansäuremethylester (57)

Analog 103 (Beispiel 54) wurden aus 1.98 g (5 mmol) 16, 1.27 g (10 mmol) Oxalylchlorid und 20 ml abs. Methanol 1.1 g (57%) 57 erhalten.

### Beispiel 58

### 2,2-Dichlor-13-(cyclohexyl-oxy)-tridecansäure (58)

### 1-Brom-11-(cyclohexyl-oxy)-undecan (107)

Analog 74 (Beispiel 22) wurden aus 1.22 g (30 mmol) Natriumhydrid (60%ig in Weißöl) und 2.92 g (29.2 mmol) Cyclohexanol und 18.85 g (60 mmol) 1,11-Dibromundecan (Aldrich) 5.6 g (58%) 107 als blaßgelbes Öl erhalten.
Wie in Beispiel 9 wurden aus 5.41 g (16.23 mmol) 107 und 6.28 g (48.96 mmol) Dichloressigsäure nach Flashchromatographie an Kieselgel 1.25 g (21%) 58 als farbloses Öl erhalten.

Das aus 58 und pulverisierter NaOH in Ethanol erhaltene Natriumsalz schmolz bei 75 - 78°C.

### Beispiel 59

### 2,2-Dichlor-14-(4-chlorphenvl-sulfonylamino)-tetradecansäure (59)

### 11-Cyano-undecanol-(1) (108)

Zu einer Lösung von 25.1 g (0.1 mol) 11-Bromundecanol-(1) in 50 ml DMSO wurden bei 100°C 7.89 (0.12 mol) Kaliumcyanid gelöst in 20 ml Wasser innerhalb einer Stunde zugetropft. Nach 6 Stunden Rühren unter Rückfluß wurde abgekühlt, mit 100 ml Wasser verdünnt und ausgeethert. Nach Waschen der org. Phase wurde getrocknet (MgSO₄) und konzentriert. Man erhielt nach Flashromatographie an Kieselgel (Heptan/Essigester 5:1) 11.1 g (53%) farblose Kristalle vom Schmp. 34-35°C.

### 12-Amino-dodecanol-(1) (109)

In einer Hochdruckhydrierapparatur wurden 11.0 g (52 mmol) 108 in 150 ml Methanol mit 5.0 g Raney-Nickel Katalysator unter Zusatz von 50 ml flüssigem Ammoniak 8 Stunden bei 80°C / 120 bar hydriert. Nach Filtration, Konzentrieren und Trocknung im Hochvakuum verblieben 10.3 g (98%) hellgelbes Öl 109.

### 12-(4-Chlorphenyl-sulfonylamino)-dodecanol-(1) (110)

In 200 ml Pyridin wurden 10 g (46.4 mmol) 109 gelöst und bei 30°C 9.8 g (46.4 mmol) p-Chlorbenzolsulfonsäurechlorid zugegeben. Man rührte über Nacht bei Raumtemperatur, entfernte durch Destillation die Hauptmenge Pyridin und verteilte den Rückstand zwischen Wasser und Ether. Nach Eindampfen der organischen Phase erhielt man 5.9 g (34%) 110 vom Schmp. 90 - 92°C.

### 1-Brom-12-(4-chlorphenyl-sulfonylamino)-dodecan (111)

5.62 (15 mmol) 110 wurden mit 15 ml Phosphortribromid 2 Stunden auf 40°C erwärmt, abgekühlt, in Eiswasser gegossen und mit Ether extrahiert. Nach Trocknen (MgSO4) und konzentrieren erhielt man 5.16 g (78%) 111 vom Schmp. 70 - 73°C.

Analog zu Beispiel 9 wurden aus 1.3 g (3 mmol) 101 und 2,3 g (18 mmol) Dichloressigsäure nach Flashchromatographie an Kieselgel 0.6 g (46%) 59 als farbloses Kristallisat vom Schmp. 70°C erhalten. Das aus 59 und pulverisierter NaOH in Ethanol erhaltene Natriumsalz schmolz bei 150 - 152°C.

### Beispiel 60

### Pharmakologischer Testbericht:

Rattenhepatozyten in Kultur sind für Untersuchungen des zellulären Stoffwechsels geeignet. Diese Primärkulturen bieten den Vorteil, daß mehrere Substanzen vergleichend in einem nichtproliferierenden also primär durch metabolische Vorgänge bestimmten System untersucht werden können.

Rattenhepatozyten wurden durch rezirkulierende Collagenaseperfusion isoliert und in Schrägbodenröhrchen kultiviert. Der Insulin-stimulierte ¹⁴C-Acetateinbau in Triglyceride (TG) wurde in Gegenwart und Abwesenheit von Testsubstanzen untersucht.

**Tabelle:**

| | |
|---|---|
| Wirkung von 2,2-Dichloralkancarbonsäuren auf den Einbau von ¹⁴C-Acetat in Triglyceride (TG) in primären Monolayerkulturen von Leberzellen aus männlichen Sprague-Dawley-Ratten während einer Inkubationszeit von 48 Stunden in Serumfreiem Dulbecco MEM. Angegeben sind die Unterschiede zu Lösungsmittelbehandelten Kontrollen (DMSO 0,1% v/v) in Prozent (4 Kulturschalen von 2 Präparationen). | |

| Substanzen (Verb. d. Bsp.) | %-Erhöhung des ¹⁴C-Acetateinbaus in ¹⁴C-TG |
|---|---|
| Bsp. 10 | 23 |
| Bsp. 32 | 51 |
| Bsp. 26 | 37 |
| Bsp. 9 | 31 |
| Bsp. 41 | 27 |
| Bsp. 11 | 28 |
| Bsp. 16 | 36 |
| Bsp. 46 | 28 |
| Bsp. 47 | 19 |

Die hier genannten 2,2 Dichloralkancarbonsäuren führen zu einer deutlichen Verstärkung des Insulin-stimulierten ¹⁴C-Acetateinbaus in Triglyceride. Dies ist ein Hinweis, daß die erfindungsgemäßen Verbindungen antidiabetisch wirken. Dieser Effekt kommt vor allem in einer starken Reduktion der TG de novo Synthese zum Ausdruck.

## Patentansprüche

1. Arzneimittel, enthaltend mindestens eine Verbindung der Formel I 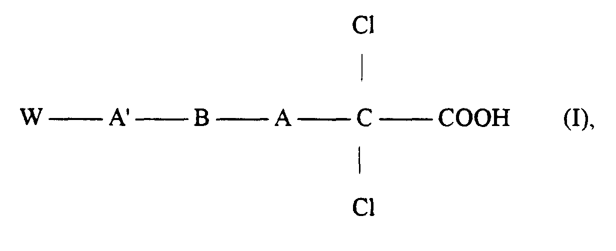 in welcher
A eine Alkylenkette mit 5 - 20 Kohlenstoffatomen,
A' ein Valenzstrich, eine Vinylen- oder Acetylengruppe oder eine Alkylenkette mit 1 - 10 Kohlenstoffatomen,
B ein Valenzstrich, eine Methylengruppe, Schwefel, Sauerstoff oder die Gruppe NR¹,
wobei
R¹ Wasserstoff, Benzyl, Phenyl oder ein C₁-C₄-Alkylrest sein kann,
eine Carbonyl-, Sulfonamid-, Sulfoxid- oder Sulfongruppe, eine E- oder Z-Vinylen- oder eine Acetylengruppe, eine CR²R³-Gruppe,
wobei
R² Wasserstoff, ein C₁-C₄-Alkylrest oder Phenyl,
R³ ein C₁-C₄-Alkylrest, Benzyl, Phenyl, Hydroxy oder eine Gruppe NR⁴R⁵,
worin
R⁴ Wasserstoff, Benzyl, Phenyl oder ein C₁-C₄-Alkylrest und
R⁵ Wasserstoff oder ein C₁-C₄-Alkylrest sein kann,
eine Gruppe Y-Z-Y,
wobei
Y Schwefel oder Sauerstoff,
Z eine Alkylkette (CH₂)ₙ und n 1-5 sein kann,
bedeutet, und
W ein Halogenatom; eine Cyano- oder Rhodanogruppe, Aminocarbonyl; ein Methyl, Isopropyl oder tert-Butylrest; ein C₃-C₈-Cycloalkylrest, der unsubstituiert oder durch Phenyl oder C₁ - C₄-Alkyl substituiert sein kann; ein Cyclohexenyl- oder Cyclopentenylrest, ein Phenylring, der substituiert sein kann durch einen oder eine beliebige Kombinationen der folgenden Substituenten: C₁-C₄-Alkyl, C₁-C₄-Alkoxy, C₁-C₄-Alkylthio, C₁-C₄-Alkylsulfinyl, C₁-C₄-Alkylsulfonyl, Trifluormethyl, Nitro, Amino, Hydroxy, Cyano, Mercapto, Sulfonamino, Acetylamino. Carboxy, Phenoxy, Benzyloxy, Phenyl, Benzoyl, Carboxy-C₁-C₄-alkyl, Methylendioxy, Ethylendioxy, Fluor, Chlor, Brom, Iod, Carboxymethoxy, Carboxyethoxy, Acetoxy, Acetyl, Propionyl, eine Gruppe NR⁶R⁷, wobei R⁶ Wasserstoff, C₁-C₄-Alkyl, Benzyl und R⁷ Wasserstoff C₁-C₄-Alkyl, Benzyl, Phenyl oder Benzoyl ist, wobei die jeweiligen aromatischen Ringe unsubstituiert oder ein- oder mehrfach durch Halogen, Hydroxy oder C₁-C₄-Alkoxy substituiert sein können, desweiteren ein α-oder β-Naphthylring, der durch Methyl, Hydroxy, Methoxy, Carboxy, Methoxycarbonyl, Ethoxycarbonyl, Cyano, Acetyl, Chlor oder Brom substituiert sein kann oder ein Tetrahydronaphthylrest,
bedeuten,
sowie deren physiologisch verträgliche Salze oder Ester und optische Isomeren.

2. Arzneimittel nach Anspruch 1, bei denen in Formel I
A eine Alkylenkette mit 8 - 14, vorzugsweise 10 - 12 Kohlenstoffatomen,
A' ein Valenzstrich, Vinylen oder Acetylen,
B Valenzstrich, eine Methylengruppe, Sauerstoff, Schwefel, Sulfoxid, Sulfonamid oder Sulfonyl und
W ein C₃-C₈-Cycloalkyl oder ein gegebenenfalls substituierter Phenylrest, insbesondere 4-Chlorphenyl, 4-Methylthiophenyl, 4-C₁-C₄-Alkylphenyl, 4-Methylsulfonylphenyl bedeutet.

3. Verwendung von Verbindungen der Formel I gemäß einem der Ansprüche 1 oder 2 zur Herstellung von Arzneimitteln zur Behandlung des Diabetes mellitus.

4. Neue Verbindungen der Formel I 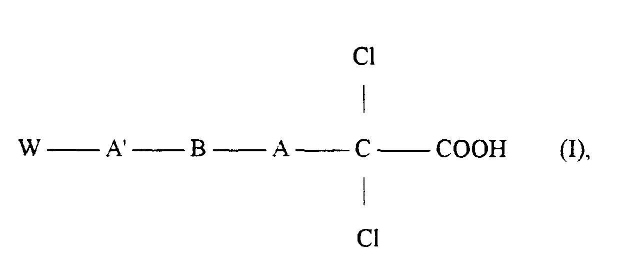 in welcher
A eine Alkylenkette mit 5 - 20 Kohlenstoffatomen,
A' ein Valenzstrich, eine Vinylen- oder Acetylengruppe oder eine Alkylenkette mit 1 - 10 Kohlenstoffatomen,
B ein Valenzstrich, eine Methylengruppe, Schwefel, Sauerstoff oder die Gruppe NR¹,
wobei
R¹ Wasserstoff, Benzyl, Phenyl oder ein C₁-C₄-Alkylrest sein kann,
eine Carbonyl-, Sulfonamid-, Sulfoxid- oder Sulfongruppe, eine E- oder Z-Vinylen- oder eine Acetylengruppe, eine CR²R³-Gruppe,
wobei
R² Wasserstoff, ein C₁-C₄-Alkylrest oder Phenyl,
R³ ein C₁-C₄-Alkylrest, Benzyl, Phenyl, Hydroxy oder eine Gruppe NR⁴R⁵,
worin
R⁴ Wasserstoff, Benzyl, Phenyl oder ein C₁-C₄-Alkylrest und
R⁵ Wasserstoff oder ein C₁-C₄-Alkylrest sein kann.
eine Gruppe Y-Z-Y,
wobei
Y Schwefel oder Sauerstoff.
Z eine Alkylkette (CH₂)ₙ und n 1-5 sein kann.
bedeutet, und
W Brom, eine Cyano- oder Rhodanogruppe, Aminocarbonyl; ein C₃-C₈-Cycloalkylrest. der unsubstituiert oder durch Phenyl oder C₁-C₄-Alkyl substituiert sein kann; ein Cyclohexenyl- oder Cyclopentenylrest, ein Phenylring, der substituiert sein kann durch einen oder eine beliebige Kombinationen der folgenden Substituenten: C₁-C₄-Alkyl, C₁-C₄-Alkoxy, C₁-C₄-Alkylthio, C₁-C₄-Alkylsulfinyl, C₁-C₄-Alkylsulfonyl, Trifluormethyl, Nitro, Amino, Hydroxy, Cyano, Mercapto, Sulfonamino, Acetylamino, Carboxy, Phenoxy, Benzyloxy, Phenyl, Benzoyl, Carboxy-C₁-C₄-alkyl, Methylendioxy, Ethylendioxy, Fluor, Chlor, Brom, Iod, Carboxymethoxy, Carboxyethoxy, Acetoxy, Acetyl, Propionyl, eine Gruppe NR⁶R⁷, wobei R⁶ Wasserstoff. C₁-C₄-Alkyl, Benzyl und R⁷ Wasserstoff C₁-C₄-Alkyl, Benzyl, Phenyl oder Benzoyl ist, wobei die jeweiligen aromatischen Ringe unsubstituiert oder ein- oder mehrfach durch Halogen, Hydroxy oder C₁-C₄-Alkoxy substituiert sein können, desweiteren ein α- oder β-Naphthylring, der durch Methyl, Hydroxy, Methoxy, Carboxy, Methoxycarbonyl, Ethoxycarbonyl, Cyano, Acetyl, Chlor oder Brom substituiert sein kann oder ein Tetrahydronaphthylrest,
bedeuten,
sowie deren physiologisch verträglichen Salze, Ester und optisch aktiven Formen.

5. Neue Verbindungen nach Anspruch 4, bei denen in Formel I
A eine Alkylenkette mit 8-14, vorzugsweise 10-12 Kohlenstoffatomen,
A' ein Valenzstrich, eine Vinylen- oder Acetylengruppe
B ein Valenzstrich, eine Methylengruppe, Schwefel, Sauerstoff, Sulfoxid oder Sulfonyl
W ein C₃-C₈-Cycloalkylrest oder ein gegebenfalls substituierter Phenylrest, insbesondere 4-Chlorphenyl, 4-Methylthiophenyl, 4-C₁-C₄-Alkylphenyl, 4-Methylsulfonyl
bedeutet,
sowie deren physiologisch verträglichen Salze, Ester und optisch aktive Formen

6. Verbindung nach einem der Ansprüche 4 oder 5, 2,2-Dichlor-12-(4-chlorphenyl)-dodecansäure sowie deren physiologisch verträglichen Salze, Ester und optisch aktiven Formen.

7. Verfahren zur Herstellung von Verbindungen gemäß einem der Ansprüche 4 bis 6, **dadurch gekennzeichnet, daß** man nach an sich bekannten Methoden eine Verbindung der Formel II
X―A―B―A'―W (II),
in der A, B, A' und W die angegebene Bedeutung haben und X Halogen bedeutet,
mit Dichloressigsäure oder einem Ester der Dichloressigsäure in Gegenwart von starken Basen umsetzt und anschließend gewünschtenfalls die erhaltenen Verbindungen der Formel I in andere Verbindungen der Formel I durch Oxidation, Hydrierung oder Verseifung überführt sowie gegebenenfalls freie Säure in Ester oder Salze überführt.

## Claims

1. Medicament containing at least one compound of formula I 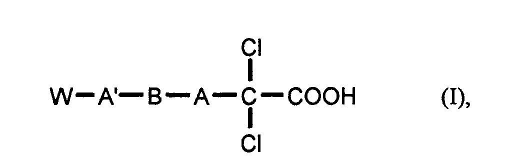 in which
A signifies an alkylene chain with 5-20 carbon atoms,
A' signifies a valency bond, a vinylene or acetylene group or an alkylene chain with 1-10 carbon atoms,
B signifies a valency bond, a methylene group, sulphur, oxygen or the NR¹ group,
in which
R¹ can be hydrogen, benzyl, phenyl or a C₁-C₄ alkyl residue,
a carbonyl, sulphonamide, sulphoxide or sulphone group, an E- or Z-vinylene or an acetylene group, a CR²R³ group,
in which
R² can be hydrogen, a C₁-C₄ alkyl residue or phenyl,
R³ can be a C₁-C₄ alkyl residue, benzyl, phenyl, hydroxy or a NR⁴R⁵ group,
in which
R⁴ can be hydrogen, benzyl, phenyl or a C₁-C₄ alkyl residue and
R⁵ can be hydrogen or a C₁-C₄ alkyl residue,
a group Y-Z-Y,
in which
Y can be sulphur or oxygen,
Z can be an alkyl chain (CH₂)ₙ and n can be 1-5,
and
w signifies a halogen atom; a cyano or thiocyanato group; aminocarbonyl; a methyl, isopropyl or tert-butyl residue; a C₃-C₈ cycloalkyl residue which can be unsubstituted or substituted by phenyl or C₁-C₄ alkyl; a cyclohexenyl or cyclopentenyl residue, a phenyl ring which can be substituted by one or an optional combination of the following substituents: C₁-C₄ alkyl, C₁-C₄ alkoxy, C₁-C₄ alkylthio, C₁-C₄ alkylsulphinyl, C₁-C₄ alkylsulphonyl, trifluoromethyl, nitro, amino, hydroxy, cyano, mercapto, sulphonamino, acetylamino, carboxy, phenoxy, benzyloxy, phenyl, benzoyl, carboxy-C₁-C₄-alkyl, methylenedioxy, ethylenedioxy, fluorine, chlorine, bromine, iodine, carboxymethoxy, carboxyethoxy, acetoxy, acetyl, propionyl, a NR⁶R⁷ group in which R⁶ signifies hydrogen, C₁-C₄ alkyl, benzyl and R⁷ signifies hydrogen, C₁-C₄ alkyl, benzyl, phenyl or benzoyl, whereby the respective aromatic rings can be unsubstituted or mono- or multiply-substituted by halogen, hydroxy or C₁-C₄ alkoxy, furthermore an α- or β-naphthyl ring which can be substituted by methyl, hydroxy, methoxy, carboxy, methoxycarbonyl, ethoxycarbonyl, cyano, acetyl, chlorine or bromine or a tetrahydronaphthyl residue,
as well as physiologically compatible salts or esters and optical isomers thereof.

2. Medicament according to claim 1, wherein in formula I
A signifies an alkylene chain with 8-14, preferably 10-12, carbon atoms,
A' signifies a valency bond, vinylene or acetylene,
B signifies a valency bond, a methylene group, oxygen, sulphur, sulphoxide, sulphonamide or sulphonyl and
w signifies a C₃-C₈-cycloalkyl or an optionally substituted phenyl residue, especially 4-chlorphenyl, 4-methylthiophenyl, 4-C₁-C₄-alkylphenyl, 4-methylsulphonylphenyl.

3. Use of compounds of formula I in accordance with one of claims 1 or 2 for the production of medicaments for the treatment of diabetes mellitus.

4. Novel compounds of formula I 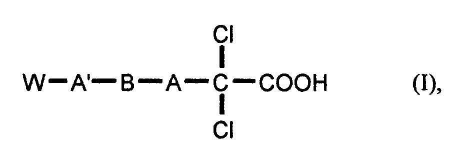 in which
A signifies an alkylene chain with 5-20 carbon atoms,
A' signifies a valency bond, a vinylene or acetylene group or an alkylene chain with 1-10 carbon atoms,
B signifies a valency bond, a methylene group, sulphur, oxygen or the NR¹ group,
in which
R¹ can be hydrogen, benzyl, phenyl or a C₁-C₄ alkyl residue,
a carbonyl, sulphonamide, sulphoxide or sulphone group, an E- or Z-vinylene or an acetylene group, a CR²R³ group,
in which
R² can be hydrogen, a C₁-C₄ alkyl residue or phenyl,
R³ can be a C₁-C₄ alkyl residue, benzyl, phenyl, hydroxy or a NR⁴R⁵ group,
in which
R⁴ can be hydrogen, benzyl, phenyl or a C₁-C₄ alkyl residue and
R⁵ can be hydrogen or a C₁-C₄ alkyl residue,
a group Y-Z-Y,
in which
Y can be sulphur or oxygen,
Z can be an alkyl chain (CH₂)ₙ and n can be 1-5,
and
W signifies bromine; a cyano or thiocyanato group; aminocarbonyl; a C₃-C₈ cycloalkyl residue which can be unsubstituted or substituted by phenyl or C₁-C₄ alkyl; a cyclohexenyl or cyclopentenyl residue, a phenyl ring which can be substituted by one or an optional combination of the following substituents: C₁-C₄ alkyl, C₁-C₄ alkoxy, C₁-C₄ alkylthio, C₁-C₄ alkylsulphinyl, C₁-C₄ alkylsulphonyl, trifluoromethyl, nitro, amino, hydroxy, cyano, mercapto, sulphonamino, acetylamino, carboxy, phenoxy, benzyloxy, phenyl, benzoyl, carboxy-C₁-C₄ alkyl, methylenedioxy, ethylenedioxy, fluorine, chlorine, bromine, iodine, carboxymethoxy, carboxyethoxy, acetoxy, acetyl, propionyl, a NR⁶R⁷ group in which R⁶ is hydrogen, C₁-C₄ alkyl, benzyl and R⁷ is hydrogen, C₁-C₄ alkyl, benzyl, phenyl or benzoyl, whereby the respective aromatic rings can be unsubstituted or mono- or multiply-substituted by halogen, hydroxy or C₁-C₄ alkoxy, furthermore an α- or β-naphthyl ring which can be substituted by methyl, hydroxy, methoxy, carboxy, methoxycarbonyl, ethoxycarbonyl, cyano, acetyl, chlorine or bromine or a tetrahydronaphthyl residue,
as well as physiologically compatible salts, esters and optically active forms thereof.

5. Novel compounds according to claim 4, wherein in formula I
A signifies an alkylene chain with 8-14, preferably 10-12, carbon atoms,
A' signifies a valency bond, a vinylene or acetylene group,
B signifies a valency bond, a methylene group, sulphur, oxygen, sulphoxide or sulphonyl,
W signifies a C₃-C₈-cycloalkyl residue or an optionally substituted phenyl residue, especially 4-chlorphenyl, 4-methylthiophenyl, 4-C₁-C₄-alkylphenyl, 4-methylsulphonyl,
as well as physiologically compatible salts, esters and optically active forms thereof.

6. Compound of formula I in accordance with one of claims 4 or 5, 2,2-dichloro-12-(4-chlorophenyl)-dodecanoic acid as well as physiologically compatible salts, esters and optically active forms thereof

7. Process for the manufacture of compounds in accordance with one of claims 4 to 6, **characterized in that** a compound of formula II
X―A―B―A'―W (II),
in which A, B, A' and W have the given significance and X signifies halogen,
is reacted according to methods known per se with dichloroacetic acid or a dichloroacetic acid ester in the presence of strong bases and subsequently, if desired, the compounds of formula I obtained are converted into other compounds of formula I by oxidation, hydrogenation or saponification and, if desired, free acids are converted into esters or salts.

## Revendications

1. Médicaments contenant au moins un composé de formule I 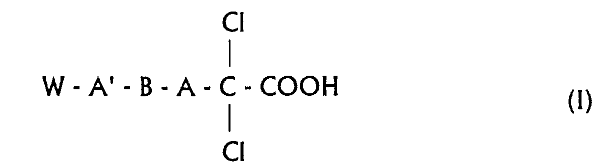 dans laquelle
A est une chaîne alkylène ayant de 5 à 20 atomes de carbone,
A' est un trait de valence, un groupe vinylène ou acétylène, ou une chaîne alkylène ayant de 1 à 10 atomes de carbone,
B est un trait de valence, un groupe méthylène, un atome de soufre ou d'oxygène, ou le groupe NR¹, dans lequel R¹ peut être un atome d'hydrogène, un groupe benzyle ou phényle, ou un radical alkyle en C₁-C₄,
un groupe carbonyle, sulfonamido, sulfoxyde ou sulfonyle,
un groupe E- ou Z-vinylène ou un groupe acétylène,
un groupe CR²R³ dans lequel R² peut être un atome d'hydrogène ou un radical alkyle en C₁-C₄ ou phényle, R³ peut être un radical alkylène en C₁-C₄, benzyle, phényle, hydroxy, ou un groupe NR⁴R⁵ dans lequel R⁴ peut être un atome d'hydrogène ou un groupe benzyle, phényle, ou un radical alkyle en C₁-C₄, et R⁵ peut être un atome d'hydrogène ou un radical alkyle en C₁-C₄,
un groupe Y-Z-Y dans lequel Y peut être un atome d'oxygène ou de soufre et Z peut être une chaîne alkyle (CH₂)ₙ, et n peut valoir de 1 à 5, et
W est un atome d'halogène ; un groupe cyano ou rhodano, amino-carbonyle ; un radical méthyle, isopropyle ou tert-butyle ; un radical cycloalkyle en C₃-C₈, qui peut être non-substitué ou substitué par des substituants phényle ou alkyle en C₁-C₄ ; un radical cyclohexényle ou cyclopentényle, un noyau phényle, qui peut être substitué par l'un des substituants suivants ou par une combinaison quelconque des substituants suivants : alkyle en C₁-C₄, alcoxyen C₁-C₄, alkylthio en C₁-C₄, alkylsulfinyle en C₁-C₄, alkylsulfonyle en C₁-C₄, trifluorométhyle, nitro, amino, hydroxy, cyano, mercapto, sulfonamino, acétylamino, carboxy, phénoxy, benzyloxy, phényle, benzoyle, carboxy-(alkyle en C₁-C₄), méthylènedioxy, éthylènedioxy, fluoro, chloro, bromo, iodo, carboxyméthoxy, carboxyéthoxy, acétoxy, acétyle, propionyle, un groupe NR⁶R⁷ dans lequel R⁶ est un atome d'hydrogène ou un radical alkyle en C₁-C₄ ou le groupe benzyle et R⁷ est un atome d'hydrogène ou un radical alkyle en C₁-C₄ ou le groupe benzyle, phényle ou benzoyle, où les noyaux aromatiques peuvent être non-substitués ou une ou plusieurs fois substitués par des substituants halogéno, hydroxy ou alcoxy en C₁-C₄, et en outre un noyau α- ou β-naphtyle, qui peut être substitué par des substituants méthyle, hydroxy, méthoxy, carboxy, méthoxycarbonyle, éthoxycarbonyle, cyano, acétyle, chloro ou bromo, ou encore un radical tétrahydronaphtyle,
ainsi que leurs sels ou esters et isomères optiques physiologiquement tolérés.

2. Médicaments selon la revendication 1, dans lesquels, dans la formule I
A est une chaîne alkylène ayant de 8 à 14 et de préférence de 10 à 12 atomes de carbone,
A' est un trait de valence ou un groupe vinylène ou acétylène,
B est un trait de valence, un groupe méthylène, un atome d'oxygène ou de soufre, ou un groupe sulfoxyde, sulfonamido ou sulfonyle, et
W est un radical cycloalkyle en C₃-C₈ ou un radical phényle éventuellement substitué, en particulier un groupe 4-chlorophényle, 4-méthylthiophényle, 4-(alkyle en C₁-C₄)-phényle, 4-méthylsulfonylphényle.

3. Utilisation de composés de formule I selon l'une des revendications 1 ou 2 pour préparer des médicaments destinés au traitement du diabète sucré.

4. Nouveaux composés de formule I 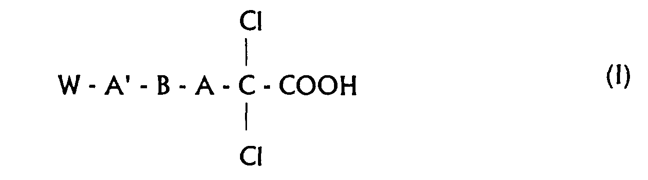 dans laquelle
A est une chaîne alkylène ayant de 5 à 20 atomes de carbone,
A' est un trait de valence, un groupe vinylène ou acétylène, ou une chaîne alkylène ayant de 1 à 10 atomes de carbone,
B est un trait de valence, un groupe méthylène, un atome de soufre ou d'oxygène, ou le groupe NR¹, dans lequel R¹ peut être un atome d'hydrogène, un groupe benzyle ou phényle, ou un radical alkyle en C₁-C₄,
un groupe carbonyle, sulfonamido, sulfoxyde ou sulfonyle,
un groupe E- ou Z-vinylène ou un groupe acétylène,
un groupe CR²R³ dans lequel R² peut être un atome d'hydrogène ou un radical alkyle en C₁-C₄ ou phényle, R³ peut être un radical alkylène en C₁-C₄, benzyle, phényle, hydroxy, ou un groupe NR⁴R⁵ dans lequel R⁴ peut être un atome d'hydrogène ou un groupe benzyle, phényle, ou un radical alkyle en C₁-C₄, et R⁵ peut être un atome d'hydrogène ou un radical alkyle en C₁-C₄,
un groupe Y-Z-Y dans lequel Y peut être un atome d'oxygène ou de soufre et Z peut être une chaîne alkyle (CH₂)ₙ, et n peut valoir de 1-5, et
W est un groupe bromo, cyano ou rhodano, un groupe aminocarbonyle ; un radical cycloalkyle en C₃-C₈, qui peut être non-substitué ou substitué par des substituants phényle ou alkyle en C₁-C₄ ; un radical cyclohexényle ou cyclopentényle, un noyau phényle, qui peut être substitué par l'un des substituants suivants ou par une combinaison quelconque des substituants suivants : alkyle en C₁-C₄, alcoxy en C₁-C₄, alkylthio en C₁-C₄, alkylsulfinyle en C₁-C₄, alkylsulfonyle en C₁-C₄, trifluorométhyle, nitro, amino, hydroxy, cyano, mercapto, sulfonamino, acétylamino, carboxy, phénoxy, benzyloxy, phényle, benzoyle, carboxy-(alkyle en C₁-C₄), méthylènedioxy, éthylènedioxy, fluoro, chloro, bromo, iodo, carboxyméthoxy, carboxyéthoxy, acétoxy, acétyle, propionyle, un groupe NR⁶R⁷ dans lequel R⁶ est un atome d'hydrogène ou un radical alkyle en C₁-C₄ ou le groupe benzyle et R⁷ est un atome d'hydrogène ou un radical alkyle en C₁-C₄ ou le groupe benzyle, phényle ou benzoyle, où les noyaux aromatiques peuvent être non-substitués ou une ou plusieurs fois substitués par des substituants halogéno, hydroxy ou alcoxy en C₁-C₄, et en outre un noyau α- ou β-naphtyle, qui peut être substitué par des substituants méthyle, hydroxy, méthoxy, carboxy, méthoxycarbonyle, éthoxycarbonyle, cyano, acétyle, chloro ou bromo, ou encore un radical tétrahydronaphtyle,
ainsi que leurs sels ou esters et formes optiquement actives physiologiquement tolérés.

5. Nouveaux composés selon la revendication 4, dans lesquels, dans la formule I
A est une chaîne alkylène ayant de 8 à 14 et de préférence de 10 à 12 atomes de carbone,
A' est un trait de valence ou un groupe vinylène ou acétylène,
B est un trait de valence, un groupe méthylène, un atome de soufre ou d'oxygène ou un groupe sulfoxyde ou sulfonyle, et
W est un radical cycloalkyle en C₃-C₈ ou un radical phényle éventuellement substitué, en particulier un groupe 4-chlorophényle, 4-méthylthiophényle, 4-(alkyle en C₁-C₄)-phényle, 4-méthylsulfonyle,
ainsi que leurs sels, esters et formes optiquement actives physiologiquement tolérés.

6. Composé selon l'une des revendications 4 ou 5, l'acide 2,2-dichloro-12-(4-chlorophényl)-dodécanoïque, ainsi que ses sels, esters et formes optiquement actives physiologiquement tolérés.

7. Procédé de préparation de composés selon l'une des revendications 4 à 6, **caractérisé en ce qu'**on fait réagir par des procédés connus en soi un composé de formule II
X-A-B-A'-W (II)
dans laquelle A, B, A' et W ont les significations données ci-dessus et X est un halogène,
avec de l'acide dichloracétique ou un ester de l'acide dichloracétique en présence de bases fortes, puis, si on le souhaite, on convertit les composés obtenus de formule I en d'autres composés de formule I par oxydation, hydrogénation ou saponification, et éventuellement on convertit l'acide libre en esters ou en sels.
